# EUROPEAN PATENT APPLICATION

(11) **EP 1 444 995 A1**
(43) Date of publication of application: **11.08.2004**
(21) Application number: 03290305.6
(22) Date of filing: 06.02.2003
(51) Int. Cl.: A61L 31/04, A61L 31/10, A61L 33/12, C07K 14/50

(54) **FGF-2 derived proteins for the preparation of biomaterials or medical devices such as stents**

(71) Applicant: INSTITUT NATIONAL DE LA SANTE ET DE LA RECHERCHE MEDICALE (INSERM), 75654 Paris Cédex 13 (FR)
(72) Inventor: Prats, Hervé, F-31400 Toulouse (FR); Laurell, Henrik, F-31400 Toulouse (FR); Bossard, Carine, F-31400 Toulouse (FR); Garmy-Susini, Barbara, F-31170 Tounefeuille (FR); Arnal, Jean-Francois, FR-31000 Toulouse (FR)
(74) Representative: Grosset-Fournier, Chantal Catherine

(57) **Abstract**

The invention relates to the use of basic fibroblast growth factor (FGF-2) derived proteins being chosen among FGF-2 mutants which are unable to interact specifically with the Translokin, for the preparation of biomaterials or devices chosen among medical prostheses, such as endovascular stents, or bypass grafts, said FGF-2 derived proteins being able to accelerate the endothelialization of said medical prostheses and/or the reendothelialization of injured blood vessels and/or generating an endothelium with macroscopic characteristics similar to a healthy endothelium and in the meantime minimizing restenosis of said medical prostheses or blood vessels.

SEQUENCE LISTING

<110>  INSERM

<120>  FGF-2 DERIVED PROTEINS FOR THE PREPARATION OF BIOMATERIALS OR MEDICAL
DEVICES SUCH AS STENTS

<130>  IFB 02 CA INS NB1A

<160>  24

<170>  PatentIn version 3.1

<210>  1
<211>  1500
<212>  DNA
<213>  Homo sapiens

<220>
<221>  CDS
<222>  (1)..(1500)
<223>

<400>  1
atg gcg gcg gcg tct gtc tct gcg gct tct ggt tct cac ttg tcg aac        48
agc ttt gct gag cca tca agg tct aat gga agc atg gtt cgg cat tct        96
tca tct cca tat gta gta tat cct tcg gat aag cct ttc ctt aat agt        144
gat cta cga cgc tcc cca agt aag cct aca ctt gcc tat cca gaa agc        192
aac agc aga gcc ata ttt tct gct ctt aag aat ctt caa gat aag att        240
cga cgc ttg gaa ctt gag agg att cag gca gaa gaa agt gtg aaa acc        288
ttg tct aga gaa aca att gaa tat aag aaa gta ctg gat gaa cag ata        336
caa gaa agg gag aat tca aag aat gag gaa tca aag cac aat caa gaa        384
ctg aca tct cag ttg tta gct gca gaa aat aaa tgc aat cta tta gaa        432
aaa caa ttg gaa tac atg cga aat atg ata aag cat gcc gaa atg gag 480
agg aca tct gtc tta gag aaa caa gtt tcc cta gaa aga gaa cga caa 528
cat gat caa aca cat gtt cag agc caa ctt gaa aaa ttg gat ctt ctt 576
gaa cag gag tat aac aaa ctt acc aca atg cag gcc ctt gca gaa aaa 624
aaa atg caa gag ttg gaa gca aaa ctc cat gaa gaa gaa cag gaa agg 672
aaa cgc atg caa gct aag gca gct gag ttg cag act ggt cta gaa aca 720
aat aga ctt atc ttt gaa gat aag gca act ccg tgt gtt ccc aat gca 768
aga aga att aaa aaa aag aag tca aaa cca cca gaa aag aaa agt tct 816
agg aac tat ttt ggt gca caa cca cat tat aga tta tgc ttg ggt gat 864
atg cca ttt gta gct ggg aag tcc aca agc cct agc cat gcc gtg gta 912
gcc aat gtt cag ctt gtc ttg cat cta atg aag caa cac agt aaa gct 960
ttg tgc aat gat cga gtc atc aac agt att cct ttg gca aag caa gta 1008
tct tca cga ggt ggt aaa agt aag aag ttg tca gta aca cct ccc tcc 1056
tcc aac ggt att aat gag gag ttg tca gaa gtc tta cag act tta cag 1104
gat gaa ttt ggg caa atg agc ttt gat cac cag cag ctt gca aaa ctt 1152
atc cag gag tcg cca acc gtt gaa ctg aaa gac aag ttg gag tgt gaa 1200
ttg gag gca tta gtg gga agg atg gaa gca aaa gcc aac caa ata act 1248
aaa gtt cga aaa tac caa gcc cag ctg gag aaa cag aag tta gag aag 1296
cag aag aag gaa tta aaa gct acc aaa aag act ctt gat gaa gaa gga 1344
aac agc agc agc cgt tct gga atc aca ggg acc aca aat aag aaa gat 1392
ttt atg aaa ctg aga cct gga gaa aaa agg aga aaa aat ctt cag tta 1440
ttg aag gac atg caa agc ata cag aat tca tta caa agc agt agt ttg 1488
tgt tgg gat tac 1500

<210> 2
<211> 500
<212> PRT
<213> Homo sapiens

<400> 2

<210>  3
<211>  468
<212>  DNA
<213>  Bos taurus

<220>
<221>  CDS
<222>  (1)..(468)
<223>

<400> 3
atg gct gca ggt tcc atc act acc ctg ccg gct ctg cct gag gac ggt 48
Met Ala Ala Gly Ser Ile Thr Thr Leu Pro Ala Leu Pro Glu Asp Gly
1               5                   10                  15

ggt agc ggt gct ttt ccg cca ggc cac ttc aaa gac ccg aaa cgt ctg 96
Gly Ser Gly Ala Phe Pro Pro Gly His Phe Lys Asp Pro Lys Arg Leu
            20                  25                  30

tac tgc aaa aac ggt ggc ttc ttc ctg cgg atc cac ccg gac ggt cgt 144
Tyr Cys Lys Asn Gly Gly Phe Phe Leu Arg Ile His Pro Asp Gly Arg
        35                  40                  45

gtt gac ggc gtt cgt gaa aaa tct gac ccg cac atc aaa ctt cag ctg 192
Val Asp Gly Val Arg Glu Lys Ser Asp Pro His Ile Lys Leu Gln Leu
    50                  55                  60

cag gcg gaa gag cgt ggt gtt gta tct atc aaa ggt gtt tgc gcg aac 240
Gln Ala Glu Glu Arg Gly Val Val Ser Ile Lys Gly Val Cys Ala Asn
65                  70                  75                  80

cgg tac ctg gcg atg aaa gaa gac ggt cgt ctg ctg gcg tct aaa tgc 288
Arg Tyr Leu Ala Met Lys Glu Asp Gly Arg Leu Leu Ala Ser Lys Cys
                85                  90                  95

gtt acc gac gaa tgc ttc ttt ttc gaa cgt ctg gag tct aac aat tac 336
Val Thr Asp Glu Cys Phe Phe Phe Glu Arg Leu Glu Ser Asn Asn Tyr
            100                 105                 110

aac acc tat cgt tct cgt aaa tac tct tca tgg tat gtt gcg ctc aaa 384
Asn Thr Tyr Arg Ser Arg Lys Tyr Ser Ser Trp Tyr Val Ala Leu Lys
        115                 120                 125

cgc acg ggt cag tac aaa ctg ggt cca aaa acc ggt ccg ggt cag aaa 432
Arg Thr Gly Gln Tyr Lys Leu Gly Pro Lys Thr Gly Pro Gly Gln Lys
    130                 135                 140

gcg atc ctg ttc ctg ccg atg tct gcg aaa tct taa 468
Ala Ile Leu Phe Leu Pro Met Ser Ala Lys Ser
145                 150                 155

<210> 4
<211> 155
<212> PRT
<213> Bos taurus

<400> 4

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile His Pro Asp Gly Arg
        35                  40                  45

Val Asp Gly Val Arg Glu Lys Ser Asp Pro His Ile Lys Leu Gln Leu
    50                  55                  60

Gln Ala Glu Glu Arg Gly Val Val Ser Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Ser Asn Asn Tyr
            100                 105                 110

Asn Thr Tyr Arg Ser Arg Lys Tyr Ser Ser Trp Tyr Val Ala Leu Lys
        115                 120                 125

Arg Thr Gly Gln Tyr Lys Leu Gly Pro Lys Thr Gly Pro Gly Gln Lys
    130                 135                 140

Ala Ile Leu Phe Leu Pro Met Ser Ala Lys Ser
145                 150                 155

<210>  5
<211>  468
<212>  DNA
<213>  Homo sapiens

<220>
<221>  CDS
<222>  (1)..(468)
<223>

<400>  5
atg gca gcc ggg agc atc acc acg ctg ccc gcc ttg ccc gag gat ggc        48
ggc agc ggc gcc ttc ccg ccc ggc cac ttc aag gac ccc aag cgg ctg        96
tac tgc aaa aac ggg ggc ttc ttc ctg cgc atc cac ccc gac ggc cga       144
gtt gac ggg gtc cgg gag aag agc gac cct cac atc aag cta caa ctt       192
caa gca gaa gag aga gga gtt gtg tct atc aaa gga gtg tgt gct aac       240
cgt tac ctg gct atg aag gaa gat gga aga tta ctg gct tct aaa tgt     288
gtt acg gat gag tgt ttc ttt ttt gaa cga ttg gaa tct aat aac tac     336
aat act tac cgg tca agg aaa tac acc agt tgg tat gtg gca ctg aaa     384
cga act ggg cag tat aaa ctt gga tcc aaa aca gga cct ggg cag aaa     432
gct ata ctt ttt ctt cca atg tct gct aag agc tga                     468

<210>  6
<211>  155
<212>  PRT
<213>  Homo sapiens

<400>  6

<210>  7
<211>  468
<212>  DNA
<213>  Artificial

<220>
<223>  bovine FGF-2 mutant

<220>
<221>  CDS
<222>  (1)..(468)
<223>

<220>
<221>  misc_feature
<222>  (130)..(132)
<223>  cac or tta or ttg or ctt or ctc or cta or ctg

<220>
<221>  misc_feature
<222>  (142)..(144)
<223>  cgt or act or acc or aca or acg

<220>
<221>  misc_feature
<222>  (154)..(156)
<223>  gtt or act or acc or aca or acg

<220>
<221>  misc_feature
<222>  (162)..(162)
<223>  a or c or g or t

<220>
<221>  misc_feature
<222>  (163)..(165)
<223>  aaa or cgt or cgc or cga or cgg or aga or agg

<220>
<221>  misc_feature
<222>  (172)..(174)
<223>  ccg or caa or cag

<220>
<221>  misc_feature
<222>  (181)..(183)
<223>  aaa or caa or cag

<220>
<221> misc_feature
<222> (193)..(195)
<223> cag or tct or tcc or tca or tcg or agt or agc

<220>
<221> misc_feature
<222> (202)..(204)
<223> gag or tct or tcc or tca or tcg or agt or agc

<220>
<221> misc_feature
<222> (205)..(207)
<223> cgt or gtt or gtc or gta or gtg

<220>
<221> misc_feature
<222> (211)..(213)
<223> gtt or gaa or gag

<220>
<221> misc_feature
<222> (218)..(219)
<223> ct or at or ac

<400> 7
atg gct gca ggt tcc atc act acc ctg ccg gct ctg cct gag gac ggt 48
Met Ala Ala Gly Ser Ile Thr Thr Leu Pro Ala Leu Pro Glu Asp Gly
1               5                   10                  15

ggt agc ggt gct ttt ccg cca ggc cac ttc aaa gac ccg aaa cgt ctg 96
Gly Ser Gly Ala Phe Pro Pro Gly His Phe Lys Asp Pro Lys Arg Leu
            20                  25                  30

tac tgc aaa aac ggt ggc ttc ttc ctg cgg atc nnn ccg gac ggt nnn 144
Tyr Cys Lys Asn Gly Gly Phe Phe Leu Arg Ile Xaa Pro Asp Gly Xaa
        35                  40                  45

gtt gac ggc nnn cgt gan nnn tct gac nnn cac atc nnn ctt cag ctg 192
Val Asp Gly Xaa Arg Xaa Xaa Ser Asp Xaa His Ile Xaa Leu Gln Leu
    50                  55                  60

nnn gcg gaa nnn nnn ggt nnn gta tnn atc aaa ggt gtt tgc gcg aac 240
Xaa Ala Glu Xaa Xaa Gly Xaa Val Xaa Ile Lys Gly Val Cys Ala Asn
65                  70                  75                  80

cgg tac ctg gcg atg aaa gaa gac ggt cgt ctg ctg gcg tct aaa tgc 288
Arg Tyr Leu Ala Met Lys Glu Asp Gly Arg Leu Leu Ala Ser Lys Cys
                85                  90                  95

gtt acc gac gaa tgc ttc ttt ttc gaa cgt ctg gag tct aac aat tac 336
Val Thr Asp Glu Cys Phe Phe Phe Glu Arg Leu Glu Ser Asn Asn Tyr
            100                 105                 110

aac acc tat cgt tct cgt aaa tac tct tca tgg tat gtt gcg ctc aaa 384
Asn Thr Tyr Arg Ser Arg Lys Tyr Ser Ser Trp Tyr Val Ala Leu Lys
        115                 120                 125

cgc acg ggt cag tac aaa ctg ggt cca aaa acc ggt ccg ggt cag aaa 432
Arg Thr Gly Gln Tyr Lys Leu Gly Pro Lys Thr Gly Pro Gly Gln Lys
    130                 135                 140

gcg atc ctg ttc ctg ccg atg tct gcg aaa tct taa 468
Ala Ile Leu Phe Leu Pro Met Ser Ala Lys Ser
145                 150                 155

<210> 8
<211> 155
<212> PRT
<213> Artificial

<220>
<221> misc_feature
<222> (44)..(44)
<223> The 'Xaa' at location 44 stands for His, or Leu.

<220>
<221> misc_feature
<222> (48)..(48)
<223> The 'Xaa' at location 48 stands for Arg, or Thr.

<220>
<221> misc_feature
<222> (52)..(52)
<223> The 'Xaa' at location 52 stands for Val, or Thr.

<220>
<221> misc_feature
<222> (54)..(54)
<223> The 'Xaa' at location 54 stands for Glu, or Asp.

<220>
<221> misc_feature
<222> (55)..(55)
<223> The 'Xaa' at location 55 stands for Lys, or Arg.

<220>
<221> misc_feature
<222> (58)..(58)
<223> The 'Xaa' at location 58 stands for Pro, or Gln.

<220>
<221> misc_feature
<222> (61)..(61)
<223> The 'Xaa' at location 61 stands for Lys, or Gln.

<220>
<221> misc_feature
<222> (65)..(65)
<223> The 'Xaa' at location 65 stands for Gln, or Ser.

<220>
<221> misc_feature
<222> (68)..(68)

<223>  The 'Xaa' at location 68 stands for Glu, or Ser.

<220>
<221>  misc_feature
<222>  (69)..(69)
<223>  The 'Xaa' at location 69 stands for Arg, or Val.

<220>
<221>  misc_feature
<222>  (71)..(71)
<223>  The 'Xaa' at location 71 stands for Val, or Glu.

<220>
<221>  misc_feature
<222>  (73)..(73)
<223>  The 'Xaa' at location 73 stands for Tyr or Ser.

<220>
<223>  bovine FGF-2 mutant

<400>  8

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile Xaa Pro Asp Gly Xaa
        35                  40                  45

Val Asp Gly Xaa Arg Xaa Xaa Ser Asp Xaa His Ile Xaa Leu Gln Leu
    50                  55                  60

Xaa Ala Glu Xaa Xaa Gly Xaa Val Xaa Ile Lys Gly Val Cys Ala Asn

65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Ser Asn Asn Tyr
            100                 105                 110

Asn Thr Tyr Arg Ser Arg Lys Tyr Ser Ser Trp Tyr Val Ala Leu Lys
        115                 120                 125

Arg Thr Gly Gln Tyr Lys Leu Gly Pro Lys Thr Gly Pro Gly Gln Lys
    130                 135                 140

Ala Ile Leu Phe Leu Pro Met Ser Ala Lys Ser
145                 150                 155

<210>  9
<211>  468
<212>  DNA
<213>  Artificial

<220>
<223>  Chimera FGF-1 FGF-2

<220>
<221>  CDS
<222>  (1)..(468)
<223>

<400>  9
atg gct gca ggt tcc atc act acc ctg ccg gct ctg cct gag gac ggt        48
Met Ala Ala Gly Ser Ile Thr Thr Leu Pro Ala Leu Pro Glu Asp Gly
1               5                   10                  15

ggt agc ggt gct ttt ccg cca ggc cac ttc aaa gac ccg aaa cgt ctg        96
Gly Ser Gly Ala Phe Pro Pro Gly His Phe Lys Asp Pro Lys Arg Leu
            20                  25                  30

tac tgc aaa aac ggt ggc ttc ttc ctg cgg atc ctt ccg gat ggc aca       144
Tyr Cys Lys Asn Gly Gly Phe Phe Leu Arg Ile Leu Pro Asp Gly Thr
        35                  40                  45

gtg gat ggg aca agg gac agg agc gac cag cac att cag ctg cag ctc       192
Val Asp Gly Thr Arg Asp Arg Ser Asp Gln His Ile Gln Leu Gln Leu
    50                  55                  60

agt gcg gaa agc gtg ggg gag gtg tat ata aaa ggt gtt tgc gcg aac       240
Ser Ala Glu Ser Val Gly Glu Val Tyr Ile Lys Gly Val Cys Ala Asn
65                  70                  75                  80

cgg tac ctg gcg atg aaa gaa gac ggt cgt ctg ctg gcg tct aaa tgc       288
Arg Tyr Leu Ala Met Lys Glu Asp Gly Arg Leu Leu Ala Ser Lys Cys
                85                  90                  95

gtt acc gac gaa tgc ttc ttt ttc gaa cgt ctg gag tct aac aat tac       336
Val Thr Asp Glu Cys Phe Phe Phe Glu Arg Leu Glu Ser Asn Asn Tyr
            100                 105                 110

aac acc tat cgt tct cgt aaa tac tct tca tgg tat gtt gcg ctc aaa       384
Asn Thr Tyr Arg Ser Arg Lys Tyr Ser Ser Trp Tyr Val Ala Leu Lys
        115                 120                 125

cgc acg ggt cag tac aaa ctg ggt cca aaa acc ggt ccg ggt cag aaa       432
Arg Thr Gly Gln Tyr Lys Leu Gly Pro Lys Thr Gly Pro Gly Gln Lys
    130                 135                 140

gcg atc ctg ttc ctg ccg atg tct gcg aaa tct taa                       468
Ala Ile Leu Phe Leu Pro Met Ser Ala Lys Ser
145                 150                 155


<210>  10
<211>  155
<212>  PRT
<213>  Artificial

<220>
<223>  Chimera FGF-1 FGF-2

<400>  10

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile Leu Pro Asp Gly Thr
        35                  40                  45

Val Asp Gly Thr Arg Asp Arg Ser Asp Gln His Ile Gln Leu Gln Leu
    50                  55                  60

Ser Ala Glu Ser Val Gly Glu Val Tyr Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Ser Asn Asn Tyr
            100                 105                 110

Asn Thr Tyr Arg Ser Arg Lys Tyr Ser Ser Trp Tyr Val Ala Leu Lys
        115                 120                 125

Arg Thr Gly Gln Tyr Lys Leu Gly Pro Lys Thr Gly Pro Gly Gln Lys
    130                 135                 140

Ala Ile Leu Phe Leu Pro Met Ser Ala Lys Ser
145                 150                 155

<210>  11
<211>  468
<212>  DNA
<213>  Artificial

<220>
<223>  Chimera FGF-1 FGF-2

<220>
<221>  CDS
<222>  (1)..(468)
<223>

<400>  11
atg gct gca ggt tcc atc act acc ctg ccg gct ctg cct gag gac ggt          48
Met Ala Ala Gly Ser Ile Thr Thr Leu Pro Ala Leu Pro Glu Asp Gly
1               5                   10                  15

ggt agc ggt gct ttt ccg cca ggc cac ttc aaa gac ccg aaa cgt ctg          96
Gly Ser Gly Ala Phe Pro Pro Gly His Phe Lys Asp Pro Lys Arg Leu
            20                  25                  30

tac tgc aaa aac ggt ggc ttc ttc ctg cgg atc ctt ccg gat ggc aca         144
Tyr Cys Lys Asn Gly Gly Phe Phe Leu Arg Ile Leu Pro Asp Gly Thr
        35                  40                  45

gtg gat ggg aca agg gac agg agc gac cag cac att cag ctg cag ctt         192
Val Asp Gly Thr Arg Asp Arg Ser Asp Gln His Ile Gln Leu Gln Leu
    50                  55                  60

caa gca gaa gag aga gga gtt gtg tct atc aaa gga gtg tgt gct aac         240
Gln Ala Glu Glu Arg Gly Val Val Ser Ile Lys Gly Val Cys Ala Asn
65                  70                  75                  80

cgt tac ctg gct atg aag gaa gat gga aga tta ctg gct tct aaa tgt         288
Arg Tyr Leu Ala Met Lys Glu Asp Gly Arg Leu Leu Ala Ser Lys Cys
                85                  90                  95

gtt acg gat gag tgt ttc ttt ttt gaa cga ttg gaa tct aat aac tac         336
Val Thr Asp Glu Cys Phe Phe Phe Glu Arg Leu Glu Ser Asn Asn Tyr
            100                 105                 110

aat act tac cgg tca agg aaa tac acc agt tgg tat gtg gca ctg aaa         384
Asn Thr Tyr Arg Ser Arg Lys Tyr Thr Ser Trp Tyr Val Ala Leu Lys
        115                 120                 125

cga act ggg cag tat aaa ctt gga tcc aaa aca gga cct ggg cag aaa         432
Arg Thr Gly Gln Tyr Lys Leu Gly Ser Lys Thr Gly Pro Gly Gln Lys
    130                 135                 140

gct ata ctt ttt ctt cca atg tct gct aag agc tga                         468
Ala Ile Leu Phe Leu Pro Met Ser Ala Lys Ser
145                 150                 155


<210>  12
<211>  155
<212>  PRT
<213>  Artificial

<220>
<223>  Chimera FGF-1 FGF-2

<400>  12

Met Ala Ala Gly Ser Ile Thr Thr Leu Pro Ala Leu Pro Glu Asp Gly
1               5                   10                  15


Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile Leu Pro Asp Gly Thr
        35                  40                  45

Val Asp Gly Thr Arg Asp Arg Ser Asp Gln His Ile Gln Leu Gln Leu
    50                  55                  60

Gln Ala Glu Glu Arg Gly Val Val Ser Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Ser Asn Asn Tyr
            100                 105                 110

Asn Thr Tyr Arg Ser Arg Lys Tyr Thr Ser Trp Tyr Val Ala Leu Lys
        115                 120                 125

Arg Thr Gly Gln Tyr Lys Leu Gly Ser Lys Thr Gly Pro Gly Gln Lys
    130                 135                 140

Ala Ile Leu Phe Leu Pro Met Ser Ala Lys Ser
145                 150                 155

<210> 13
<211> 468
<212> DNA
<213> Homo sapiens

<220>
<221> CDS
<222> (1)..(468)
<223>

<400> 13
atg gct gaa ggg gaa atc acc acc ttc aca gcc ctg acc gag aag ttt 48
Met Ala Glu Gly Glu Ile Thr Thr Phe Thr Ala Leu Thr Glu Lys Phe
1               5                   10                  15

aat ctg cct cca ggg aat tac aag aag ccc aaa ctc ctc tac tgt agc 96
Asn Leu Pro Pro Gly Asn Tyr Lys Lys Pro Lys Leu Leu Tyr Cys Ser
            20                  25                  30

aac ggg ggc cac ttc ctg agg atc ctt ccg gat ggc aca gtg gat ggg 144
Asn Gly Gly His Phe Leu Arg Ile Leu Pro Asp Gly Thr Val Asp Gly
        35                  40                  45

aca agg gac agg agc gac cag cac att cag ctg cag ctc agt gcg gaa 192
Thr Arg Asp Arg Ser Asp Gln His Ile Gln Leu Gln Leu Ser Ala Glu
    50                  55                  60

agc gtg ggg gag gtg tat ata aag agt acc gag act ggc cag tac ttg     240
Ser Val Gly Glu Val Tyr Ile Lys Ser Thr Glu Thr Gly Gln Tyr Leu
65                  70                  75                  80

gcc atg gac acc gac ggg ctt tta tac ggc tca cag aca cca aat gag     288
Ala Met Asp Thr Asp Gly Leu Leu Tyr Gly Ser Gln Thr Pro Asn Glu
                85                  90                  95

gaa tgt ttg ttc ctg gaa agg ctg gag gag aac cat tac aac acc tat     336
Glu Cys Leu Phe Leu Glu Arg Leu Glu Glu Asn His Tyr Asn Thr Tyr
            100                 105                 110

ata tcc aag aag cat gca gag aag aat tgg ttt gtt ggc ctc aag aag     384
Ile Ser Lys Lys His Ala Glu Lys Asn Trp Phe Val Gly Leu Lys Lys
        115                 120                 125

aat ggg agc tgc aaa cgc ggt cct cgg act cac tat ggc cag aaa gca     432
Asn Gly Ser Cys Lys Arg Gly Pro Arg Thr His Tyr Gly Gln Lys Ala
    130                 135                 140

atc ttg ttt ctc ccc ctg cca gtc tct tct gat taa                     468
Ile Leu Phe Leu Pro Leu Pro Val Ser Ser Asp
145                 150                 155

<210>  14
<211>  155
<212>  PRT
<213>  Homo sapiens

<400>  14

Met Ala Glu Gly Glu Ile Thr Thr Phe Thr Ala Leu Thr Glu Lys Phe
1               5                   10                  15

Asn Leu Pro Pro Gly Asn Tyr Lys Lys Pro Lys Leu Leu Tyr Cys Ser
            20                  25                  30

Asn Gly Gly His Phe Leu Arg Ile Leu Pro Asp Gly Thr Val Asp Gly
        35                  40                  45

Thr Arg Asp Arg Ser Asp Gln His Ile Gln Leu Gln Leu Ser Ala Glu
    50                  55                  60

Ser Val Gly Glu Val Tyr Ile Lys Ser Thr Glu Thr Gly Gln Tyr Leu
65                  70                  75                  80

Ala Met Asp Thr Asp Gly Leu Leu Tyr Gly Ser Gln Thr Pro Asn Glu
                85                  90                  95

Glu Cys Leu Phe Leu Glu Arg Leu Glu Glu Asn His Tyr Asn Thr Tyr
            100                 105                 110

Ile Ser Lys Lys His Ala Glu Lys Asn Trp Phe Val Gly Leu Lys Lys
        115                 120                 125

Asn Gly Ser Cys Lys Arg Gly Pro Arg Thr His Tyr Gly Gln Lys Ala
    130                 135                 140

Ile Leu Phe Leu Pro Leu Pro Val Ser Ser Asp
145                 150                 155

<210>  15
<211>  468
<212>  DNA
<213>  Artificial

<220>
<223>  bovine FGF-2 mutant

<220>
<221>  CDS
<222>  (1)..(468)
<223>

<220>
<221>  misc_feature
<222>  (325)..(327)
<223>  tct or gaa or gag

<220>
<221>  misc_feature
<222>  (331)..(333)
<223>  aat or cat or cac

<220>
<221>  misc_feature
<222>  (346)..(348)
<223>  cgt or act or acc or aca or acg

<220>
<221>  misc_feature
<222>  (352)..(354)
<223>  cgt or aaa or aag

<220>
<221>  misc_feature
<222>  (358)..(360)
<223>  tac or cat or cac or gaa or gag or gct or gcc or gca or gcg

<220>
<221>  misc_feature
<222>  (361)..(363)
<223>  tct or gct or gcc or gca or gcg  or gaa or gag or aaa or aag

<220>
<221> misc_feature
<222> (364)..(366)
<223> tca or gaa or gag or aaa or aag or aat or aac


<220>
<221> misc_feature
<222> (370)..(372)
<223> tat or ttt or ttc


<220>
<221> misc_feature
<222> (376)..(378)
<223> gcg or ggt or ggc or gga or ggg


<400> 15
atg gct gca ggt tcc atc act acc ctg ccg gct ctg cct gag gac ggt 48
Met Ala Ala Gly Ser Ile Thr Thr Leu Pro Ala Leu Pro Glu Asp Gly
1               5                   10                  15

ggt agc ggt gct ttt ccg cca ggc cac ttc aaa gac ccg aaa cgt ctg 96
Gly Ser Gly Ala Phe Pro Pro Gly His Phe Lys Asp Pro Lys Arg Leu
            20                  25                  30

tac tgc aaa aac ggt ggc ttc ttc ctg cgg atc cac ccg gac ggt cgt 144
Tyr Cys Lys Asn Gly Gly Phe Phe Leu Arg Ile His Pro Asp Gly Arg
        35                  40                  45

gtt gac ggc gtt cgt gaa aaa tct gac ccg cac atc aaa ctt cag ctg 192
Val Asp Gly Val Arg Glu Lys Ser Asp Pro His Ile Lys Leu Gln Leu
    50                  55                  60

cag gcg gaa gag cgt ggt gtt gta tct atc aaa ggt gtt tgc gcg aac 240
Gln Ala Glu Glu Arg Gly Val Val Ser Ile Lys Gly Val Cys Ala Asn
65                  70                  75                  80

cgg tac ctg gcg atg aaa gaa gac ggt cgt ctg ctg gcg tct aaa tgc 288
Arg Tyr Leu Ala Met Lys Glu Asp Gly Arg Leu Leu Ala Ser Lys Cys
                85                  90                  95

gtt acc gac gaa tgc ttc ttt ttc gaa cgt ctg gag nnn aac nnn tac 336
Val Thr Asp Glu Cys Phe Phe Phe Glu Arg Leu Glu Xaa Asn Xaa Tyr
            100                 105                 110

aac acc tat nnn tct nnn aaa nnn nnn nnn tgg nnn gtt nnn ctc aaa 384
Asn Thr Tyr Xaa Ser Xaa Lys Xaa Xaa Xaa Trp Xaa Val Xaa Leu Lys
        115                 120                 125

cgc acg ggt cag tac aaa ctg ggt cca aaa acc ggt ccg ggt cag aaa 432
Arg Thr Gly Gln Tyr Lys Leu Gly Pro Lys Thr Gly Pro Gly Gln Lys
    130                 135                 140

gcg atc ctg ttc ctg ccg atg tct gcg aaa tct taa 468
Ala Ile Leu Phe Leu Pro Met Ser Ala Lys Ser
145                 150                 155

<210>  16
<211>  155
<212>  PRT
<213>  Artificial

<220>
<221>  misc_feature
<222>  (109)..(109)
<223>  The 'Xaa' at location 109 stands for Ser, or Glu.

<220>
<221>  misc_feature
<222>  (111)..(111)
<223>  The 'Xaa' at location 111 stands for Asn, or His.

<220>
<221>  misc_feature
<222>  (116)..(116)
<223>  The 'Xaa' at location 116 stands for Arg, or Thr.

<220>
<221>  misc_feature
<222>  (118)..(118)
<223>  The 'Xaa' at location 118 stands for Arg, or Lys.

<220>
<221>  misc_feature

<222>  (120)..(120)
<223>  The 'Xaa' at location 120 stands for Tyr, His, Ala, or Glu.

<220>
<221>  misc_feature
<222>  (121)..(121)
<223>  The 'Xaa' at location 121 stands for Ser, Ala, Glu, or Lys.

<220>
<221>  misc_feature
<222>  (122)..(122)
<223>  The 'Xaa' at location 122 stands for Ser, Glu, Lys, or Asn.

<220>
<221>  misc_feature
<222>  (124)..(124)
<223>  The 'Xaa' at location 124 stands for Tyr, or Phe.

<220>
<221>  misc_feature
<222>  (126)..(126)
<223>  The 'Xaa' at location 126 stands for Ala, or Gly.

<220>
<223>  bovine FGF-2 mutant

<400>  16

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile His Pro Asp Gly Arg
        35                  40                  45

Val Asp Gly Val Arg Glu Lys Ser Asp Pro His Ile Lys Leu Gln Leu
    50                  55                  60

Gln Ala Glu Glu Arg Gly Val Val Ser Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Xaa Asn Xaa Tyr
            100                 105                 110

Asn Thr Tyr Xaa Ser Xaa Lys Xaa Xaa Xaa Trp Xaa Val Xaa Leu Lys
        115                 120                 125

Arg Thr Gly Gln Tyr Lys Leu Gly Pro Lys Thr Gly Pro Gly Gln Lys
    130                 135                 140

Ala Ile Leu Phe Leu Pro Met Ser Ala Lys Ser
145                 150                 155

<210> 17
<211> 468
<212> DNA
<213> Artificial

<220>
<223> Chimera FGF-1 FGF-2

<220>
<221> CDS
<222> (1)..(468)
<223>

<400> 17
atg gca gcc ggg agc atc acc acg ctg ccc gcc ttg ccc gag gat ggc 48
Met Ala Ala Gly Ser Ile Thr Thr Leu Pro Ala Leu Pro Glu Asp Gly
1               5                   10                  15

ggc agc ggc gcc ttc ccg ccc ggc cac ttc aag gac ccc aag cgg ctg 96
Gly Ser Gly Ala Phe Pro Pro Gly His Phe Lys Asp Pro Lys Arg Leu
            20                  25                  30

tac tgc aaa aac ggg ggc ttc ttc ctg cgc atc cac ccc gac ggc cga 144
Tyr Cys Lys Asn Gly Gly Phe Phe Leu Arg Ile His Pro Asp Gly Arg
        35                  40                  45

gtt gac ggg gtc cgg gag aag agc gac cct cac atc aag cta caa ctt     192
Val Asp Gly Val Arg Glu Lys Ser Asp Pro His Ile Lys Leu Gln Leu
    50                  55                  60

caa gca gaa gag aga gga gtt gtg tct atc aaa gga gtg tgt gct aac     240
Gln Ala Glu Glu Arg Gly Val Val Ser Ile Lys Gly Val Cys Ala Asn
65                  70                  75                  80

cgt tac ctg gct atg aag gaa gat gga aga tta ctg gct tct aaa tgt     288
Arg Tyr Leu Ala Met Lys Glu Asp Gly Arg Leu Leu Ala Ser Lys Cys
                85                  90                  95

gtt acg gat gag tgt ttc ttt ttt gaa cga ttg gaa tac aac acc tat     336
Val Thr Asp Glu Cys Phe Phe Phe Glu Arg Leu Glu Tyr Asn Thr Tyr
            100                 105                 110

ata tcc aag aag cat gca gag aag aat tgg ttt gtt ggc ctc ctg aaa     384
Ile Ser Lys Lys His Ala Glu Lys Asn Trp Phe Val Gly Leu Leu Lys
        115                 120                 125

cga act ggg cag tat aaa ctt gga tcc aaa aca gga cct ggg cag aaa     432
Arg Thr Gly Gln Tyr Lys Leu Gly Ser Lys Thr Gly Pro Gly Gln Lys
    130                 135                 140

gct ata ctt ttt ctt cca atg tct gct aag agc tga                     468
Ala Ile Leu Phe Leu Pro Met Ser Ala Lys Ser
145                 150                 155


<210>  18
<211>  155
<212>  PRT
<213>  Artificial

<220>
<223>  Chimera FGF-1 FGF-2

<400>  18

Met Ala Ala Gly Ser Ile Thr Thr Leu Pro Ala Leu Pro Glu Asp Gly
1               5                   10                  15


Gly Ser Gly Ala Phe Pro Pro Gly His Phe Lys Asp Pro Lys Arg Leu
            20                  25                  30


Tyr Cys Lys Asn Gly Gly Phe Phe Leu Arg Ile His Pro Asp Gly Arg
        35                  40                  45


Val Asp Gly Val Arg Glu Lys Ser Asp Pro His Ile Lys Leu Gln Leu
    50                  55                  60


Gln Ala Glu Glu Arg Gly Val Val Ser Ile Lys Gly Val Cys Ala Asn
65                  70                  75                  80


Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Tyr Asn Thr Tyr
            100                 105                 110

Ile Ser Lys Lys His Ala Glu Lys Asn Trp Phe Val Gly Leu Leu Lys
        115                 120                 125

Arg Thr Gly Gln Tyr Lys Leu Gly Ser Lys Thr Gly Pro Gly Gln Lys
    130                 135                 140

Ala Ile Leu Phe Leu Pro Met Ser Ala Lys Ser
145                 150                 155

<210>  19
<211>  468
<212>  DNA
<213>  Artificial

<220>
<223>  human FGF-2 mutant

<220>
<221>  CDS
<222>  (1)..(468)
<223>

<220>
<221>  misc_feature
<222>  (130)..(132)
<223>  cac or tta or ttg or ctt or ctc or cta or ctg

<220>
<221>  misc_feature
<222>  (142)..(144)
<223>  cga or act or acc or aca or acg

<220>
<221>  misc_feature
<222>  (154)..(156)
<223>  gtc or act or acc or aca or acg

<220>
<221>  misc_feature
<222>  (162)..(162)
<223>  a or c or g or t

<220>
<221>  misc_feature
<222>  (163)..(165)
<223>  aag or cgt or cgc or cga or cgg or aga or agg

<220>
<221> misc_feature
<222> (172)..(174)
<223> cct or caa or cag


<220>
<221> misc_feature
<222> (181)..(183)
<223> aag or caa or cag


<220>
<221> misc_feature
<222> (193)..(195)
<223> caa or tct or tcc or tca or tcg or agt or agc


<220>
<221> misc_feature
<222> (202)..(204)
<223> gag or tct or tcc or tca or tcg or agt or agc


<220>
<221> misc_feature
<222> (205)..(207)
<223> aga or gtt or gtc or gta or gtg


<220>
<221> misc_feature
<222> (211)..(213)
<223> gtt or gaa or gag


<220>
<221> misc_feature
<222> (218)..(219)
<223> ct or at or ac


<400> 19
atg gca gcc ggg agc atc acc acg ctg ccc gcc ttg ccc gag gat ggc 48
Met Ala Ala Gly Ser Ile Thr Thr Leu Pro Ala Leu Pro Glu Asp Gly
1               5                   10                  15

ggc agc ggc gcc ttc ccg ccc ggc cac ttc aag gac ccc aag cgg ctg 96
Gly Ser Gly Ala Phe Pro Pro Gly His Phe Lys Asp Pro Lys Arg Leu
            20                  25                  30

tac tgc aaa aac ggg ggc ttc ttc ctg cgc atc nnn ccc gac ggc nnn 144
Tyr Cys Lys Asn Gly Gly Phe Phe Leu Arg Ile Xaa Pro Asp Gly Xaa
        35                  40                  45

gtt gac ggg nnn cgg gan nnn agc gac nnn cac atc nnn cta caa ctt 192
Val Asp Gly Xaa Arg Xaa Xaa Ser Asp Xaa His Ile Xaa Leu Gln Leu
    50                  55                  60

nnn gca gaa nnn nnn gga nnn gtg tnn atc aaa gga gtg tgt gct aac 240
Xaa Ala Glu Xaa Xaa Gly Xaa Val Xaa Ile Lys Gly Val Cys Ala Asn
65                  70                  75                  80

cgt tac ctg gct atg aag gaa gat gga aga tta ctg gct tct aaa tgt 288
Arg Tyr Leu Ala Met Lys Glu Asp Gly Arg Leu Leu Ala Ser Lys Cys
                85                  90                  95

gtt acg gat gag tgt ttc ttt ttt gaa cga ttg gaa tct aat aac tac 336
Val Thr Asp Glu Cys Phe Phe Phe Glu Arg Leu Glu Ser Asn Asn Tyr
            100                 105                 110

aat act tac cgg tca agg aaa tac acc agt tgg tat gtg gca ctg aaa 384
Asn Thr Tyr Arg Ser Arg Lys Tyr Thr Ser Trp Tyr Val Ala Leu Lys
        115                 120                 125

cga act ggg cag tat aaa ctt gga tcc aaa aca gga cct ggg cag aaa 432
Arg Thr Gly Gln Tyr Lys Leu Gly Ser Lys Thr Gly Pro Gly Gln Lys
    130                 135                 140

gct ata ctt ttt ctt cca atg tct gct aag agc tga 468
Ala Ile Leu Phe Leu Pro Met Ser Ala Lys Ser
145                 150                 155

<210> 20
<211> 155
<212> PRT
<213> Artificial

<220>
<221> misc_feature
<222> (44)..(44)
<223> The 'Xaa' at location 44 stands for His, or Leu.

<220>
<221> misc_feature
<222> (48)..(48)
<223> The 'Xaa' at location 48 stands for Arg, or Thr.

<220>
<221> misc_feature
<222> (52)..(52)
<223> The 'Xaa' at location 52 stands for Val, or Thr.

<220>
<221> misc_feature
<222> (54)..(54)
<223> The 'Xaa' at location 54 stands for Glu, or Asp.

<220>
<221> misc_feature
<222> (55)..(55)
<223> The 'Xaa' at location 55 stands for Lys, or Arg.

<220>
<221> misc_feature
<222> (58)..(58)
<223> The 'Xaa' at location 58 stands for Pro, or Gln.

<220>

<221>  misc_feature
<222>  (61)..(61)
<223>  The 'Xaa' at location 61 stands for Lys, or Gln.

<220>
<221>  misc_feature
<222>  (65)..(65)
<223>  The 'Xaa' at location 65 stands for Gln, or Ser.

<220>
<221>  misc_feature
<222>  (68)..(68)
<223>  The 'Xaa' at location 68 stands for Glu, or Ser.

<220>
<221>  misc_feature
<222>  (69)..(69)
<223>  The 'Xaa' at location 69 stands for Arg, or Val.

<220>
<221>  misc_feature
<222>  (71)..(71)
<223>  The 'Xaa' at location 71 stands for Val, or Glu.

<220>
<221>  misc_feature
<222>  (73)..(73)
<223>  The 'Xaa' at location 73 stands for Tyr or Ser.

<220>
<223>  human FGF-2 mutant

<400>  20

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile Xaa Pro Asp Gly Xaa
        35                  40                  45

Val Asp Gly Xaa Arg Xaa Xaa Ser Asp Xaa His Ile Xaa Leu Gln Leu
    50                  55                  60

Xaa Ala Glu Xaa Xaa Gly Xaa Val Xaa Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Ser Asn Asn Tyr
            100                 105                 110

Asn Thr Tyr Arg Ser Arg Lys Tyr Thr Ser Trp Tyr Val Ala Leu Lys
        115                 120                 125

Arg Thr Gly Gln Tyr Lys Leu Gly Ser Lys Thr Gly Pro Gly Gln Lys
    130                 135                 140

Ala Ile Leu Phe Leu Pro Met Ser Ala Lys Ser
145                 150                 155

<210>  21
<211>  468
<212>  DNA
<213>  Artificial

<220>
<223>  human FGF-2 mutant

<220>
<221>  CDS
<222>  (1)..(468)
<223>

<220>
<221>  misc_feature
<222>  (325)..(327)
<223>  tct or gaa or gag

<220>
<221>  misc_feature
<222>  (331)..(333)
<223>  aac or cat or cac

<220>
<221>  misc_feature
<222>  (346)..(348)
<223>  cgg or act or acc or aca or acg

<220>
<221>  misc_feature
<222>  (352)..(354)
<223>  agg or aaa or aag

<220>
<221>  misc_feature
<222>  (358)..(360)
<223>  tac or cat or cac or gaa or gag or gct or gcc or gca or gcg

<220>
<221>  misc_feature
<222>  (361)..(363)
<223>  acc or gct or gcc or gca or gcg or gaa or gag or aaa or aag

<220>
<221> misc_feature
<222> (364)..(366)
<223> agt or gaa or gag or aaa or aag or aat or aac

<220>
<221> misc_feature
<222> (370)..(372)
<223> tat or ttt or ttc

<220>
<221> misc_feature
<222> (376)..(378)
<223> gca or ggt or ggc or gga or ggg

<400> 21
atg gca gcc ggg agc atc acc acg ctg ccc gcc ttg ccc gag gat ggc 48
Met Ala Ala Gly Ser Ile Thr Thr Leu Pro Ala Leu Pro Glu Asp Gly
1               5                   10                  15

ggc agc ggc gcc ttc ccg ccc ggc cac ttc aag gac ccc aag cgg ctg 96
Gly Ser Gly Ala Phe Pro Pro Gly His Phe Lys Asp Pro Lys Arg Leu
            20                  25                  30

tac tgc aaa aac ggg ggc ttc ttc ctg cgc atc cac ccc gac ggc cga 144
Tyr Cys Lys Asn Gly Gly Phe Phe Leu Arg Ile His Pro Asp Gly Arg
        35                  40                  45

gtt gac ggg gtc cgg gag aag agc gac cct cac atc aag cta caa ctt 192
Val Asp Gly Val Arg Glu Lys Ser Asp Pro His Ile Lys Leu Gln Leu
    50                  55                  60

caa gca gaa gag aga gga gtt gtg tct atc aaa gga gtg tgt gct aac 240
Gln Ala Glu Glu Arg Gly Val Val Ser Ile Lys Gly Val Cys Ala Asn
65                  70                  75                  80

cgt tac ctg gct atg aag gaa gat gga aga tta ctg gct tct aaa tgt 288
Arg Tyr Leu Ala Met Lys Glu Asp Gly Arg Leu Leu Ala Ser Lys Cys
                85                  90                  95

gtt acg gat gag tgt ttc ttt ttt gaa cga ttg gaa nnn aat nnn tac 336
Val Thr Asp Glu Cys Phe Phe Phe Glu Arg Leu Glu Xaa Asn Xaa Tyr
            100                 105                 110

aat act tac nnn tca nnn aaa nnn nnn nnn tgg nnn gtg nnn ctg aaa 384
Asn Thr Tyr Xaa Ser Xaa Lys Xaa Xaa Xaa Trp Xaa Val Xaa Leu Lys
        115                 120                 125

cga act ggg cag tat aaa ctt gga tcc aaa aca gga cct ggg cag aaa 432
Arg Thr Gly Gln Tyr Lys Leu Gly Ser Lys Thr Gly Pro Gly Gln Lys
    130                 135                 140

gct ata ctt ttt ctt cca atg tct gct aag agc tga 468
Ala Ile Leu Phe Leu Pro Met Ser Ala Lys Ser
145                 150                 155

<210>  22
<211>  155
<212>  PRT
<213>  Artificial

<220>
<221>  misc_feature
<222>  (109)..(109)
<223>  The 'Xaa' at location 109 stands for Ser, or Glu.

<220>
<221>  misc_feature
<222>  (111)..(111)
<223>  The 'Xaa' at location 111 stands for Asn, or His.

<220>
<221>  misc_feature
<222>  (116)..(116)
<223>  The 'Xaa' at location 116 stands for Arg, or Thr.

<220>
<221>  misc_feature
<222>  (118)..(118)
<223>  The 'Xaa' at location 118 stands for Arg, or Lys.

<220>
<221>  misc_feature
<222>  (120)..(120)
<223>  The 'Xaa' at location 120 stands for Tyr, His, Ala, or Glu.

<220>
<221>  misc_feature
<222>  (121)..(121)
<223>  The 'Xaa' at location 121 stands for Ser, Ala, Glu, or Lys.

<220>
<221>  misc_feature
<222>  (122)..(122)
<223>  The 'Xaa' at location 122 stands for Ser, Glu, Lys, or Asn.

<220>
<221>  misc_feature
<222>  (124)..(124)
<223>  The 'Xaa' at location 124 stands for Tyr, or Phe.

<220>
<221>  misc_feature
<222>  (126)..(126)
<223>  The 'Xaa' at location 126 stands for Ala, or Gly.

<220>
<223>  human FGF-2 mutant

<400>  22

Met Ala Ala Gly Ser Ile Thr Thr Leu Pro Ala Leu Pro Glu Asp Gly
1               5                   10                  15

Gly Ser Gly Ala Phe Pro Pro Gly His Phe Lys Asp Pro Lys Arg Leu
            20                  25                  30

Tyr Cys Lys Asn Gly Gly Phe Phe Leu Arg Ile His Pro Asp Gly Arg
        35                  40                  45

Val Asp Gly Val Arg Glu Lys Ser Asp Pro His Ile Lys Leu Gln Leu
    50                  55                  60

Gln Ala Glu Glu Arg Gly Val Val Ser Ile Lys Gly Val Cys Ala Asn
65                  70                  75                  80

Arg Tyr Leu Ala Met Lys Glu Asp Gly Arg Leu Leu Ala Ser Lys Cys
                85                  90                  95

Val Thr Asp Glu Cys Phe Phe Phe Glu Arg Leu Glu Xaa Asn Xaa Tyr
            100                 105                 110

Asn Thr Tyr Xaa Ser Xaa Lys Xaa Xaa Xaa Trp Xaa Val Xaa Leu Lys
        115                 120                 125

Arg Thr Gly Gln Tyr Lys Leu Gly Ser Lys Thr Gly Pro Gly Gln Lys
    130                 135                 140

Ala Ile Leu Phe Leu Pro Met Ser Ala Lys Ser
145                 150                 155

<210> 23
<211> 465
<212> DNA
<213> Xenopus laevis

<220>
<221> CDS
<222> (1)..(465)
<223>

<400> 3
atg gcg gca ggg agc atc aca act ctg cca act gaa tcc gag gat ggg 48
Met Ala Ala Gly Ser Ile Thr Thr Leu Pro Thr Glu Ser Glu Asp Gly
1               5                   10                  15

gga aac act cct ttt tca cca ggg agt ttt aaa gac ccc aag agg ctc 96
Gly Asn Thr Pro Phe Ser Pro Gly Ser Phe Lys Asp Pro Lys Arg Leu
            20                  25                  30

tac tgc aag aac ggg ggc ttc ttc ctc agg ata aac tca gac ggg aga 144
Tyr Cys Lys Asn Gly Gly Phe Phe Leu Arg Ile Asn Ser Asp Gly Arg
        35                  40                  45

gtg gac ggg tca agg gac aaa agt gac tcg cac ata aaa tta cag cta 192
Val Asp Gly Ser Arg Asp Lys Ser Asp Ser His Ile Lys Leu Gln Leu
    50                  55                  60

caa gct gta gag cgg gga gtg gta tca ata aag gga atc act gca aat 240
Gln Ala Val Glu Arg Gly Val Val Ser Ile Lys Gly Ile Thr Ala Asn
65                  70                  75                  80

cgc tac ctt gcc atg aag gaa gat ggg aga tta aca tcg ctg agg tgt 288
Arg Tyr Leu Ala Met Lys Glu Asp Gly Arg Leu Thr Ser Leu Arg Cys
                85                  90                  95

ata aca gat gaa tgc ttc ttt ttt gaa cga ctg gaa gct aat aac tac 336
Ile Thr Asp Glu Cys Phe Phe Phe Glu Arg Leu Glu Ala Asn Asn Tyr
            100                 105                 110

aac act tac cgg tct cgg aaa tac agc agc tgg tat gtg gca cta aag 384
Asn Thr Tyr Arg Ser Arg Lys Tyr Ser Ser Trp Tyr Val Ala Leu Lys
        115                 120                 125

cga acc ggg cag tac aaa aat gga tcg agc act gga ccg gga caa aaa 432
Arg Thr Gly Gln Tyr Lys Asn Gly Ser Ser Thr Gly Pro Gly Gln Lys
    130                 135                 140

gct att tta ttt ctc cca atg tcc gca aag agc 465
Ala Ile Leu Phe Leu Pro Met Ser Ala Lys Ser
145                 150                 155

<210> 24
<211> 155
<212> PRT
<213> Xenopus laevis

<400> 4

Met Ala Ala Gly Ser Ile Thr Thr Leu Pro Thr Glu Ser Glu Asp Gly
1               5                   10                  15

Gly Asn Thr Pro Phe Ser Pro Gly Ser Phe Lys Asp Pro Lys Arg Leu
            20                  25                  30

Tyr Cys Lys Asn Gly Gly Phe Phe Leu Arg Ile Asn Ser Asp Gly Arg
        35                  40                  45

Val Asp Gly Ser Arg Asp Lys Ser Asp Ser His Ile Lys Leu Gln Leu
    50                  55                  60

Gln Ala Val Glu Arg Gly Val Val Ser Ile Lys Gly Ile Thr Ala Asn
65                  70                  75                  80

Arg Tyr Leu Ala Met Lys Glu Asp Gly Arg Leu Thr Ser Leu Arg Cys
                85                  90                  95

Ile Thr Asp Glu Cys Phe Phe Phe Glu Arg Leu Glu Ala Asn Asn Tyr
            100                 105                 110

Asn Thr Tyr Arg Ser Arg Lys Tyr Ser Ser Trp Tyr Val Ala Leu Lys
        115                 120                 125

Arg Thr Gly Gln Tyr Lys Asn Gly Ser Ser Thr Gly Pro Gly Gln Lys
    130                 135                 140

Ala Ile Leu Phe Leu Pro Met Ser Ala Lys Ser
145                 150                 155

## Description

The present invention relates to the use of basic fibroblast growth factor (FGF-2) derived proteins for the preparation of biomaterials or devices chosen among medical prostheses, such as endovascular stents, or bypass grafts, said FGF-2 derived proteins being able to accelerate the endothelialization of said medical prostheses and/or the reendothelialization of injured blood vessels and/or generating an endothelium with macroscopic characteristics similar to a healthy endothelium and in the meantime minimizing restenosis of said medical prostheses or blood vessels.

Despite therapeutic progress, the cardiovascular diseases remain the first cause of the mortality in the western countries. This progress has particularly concerned the surgical cardiology with the angioplasty and the thrombolysis as the current pillars in the treatment of the atherosclerosis. The last twenty years, these therapeutic strategies have allowed a reduction about 50 % of the mortality of the coronary disease treated in a hospital environment.

A percutaneous transluminal angioplasty (PTA) intervention consists in introducing a balloon catheter along a guidewire via a large artery of the lower or upper limbs to the occluded artery. Once positioned at the level of the occlusion, the balloon is inflated which presses the atherosclerotic plaque into the arterial wall. This results in an immediate widening of the arterial lumen and restoration of the arterial caliber. However, in the months following the intervention a renarrowing (restenosis) occur as a common (≥50%) complication. This is most often due to a constrictive remodeling of the arterial wall or the formation of a neointima (Bennett and O'Sullivan, 2001; Van Belle, *et al*., 1998).

The narrowing of the vessel lumen due to constrictive remodeling (primarily the consequence of an inflammatory reaction of the adventitia) can be prevented by the implantation of an endovascular prosthesis (stent) which mechanically prevents the constrictive reshaping (Fischman, *et al*., 1994; Serruys, *et al*., 1994).

The stent implantation does not however prevent the formation of the neointima, which is the other important factor provoking restenosis. The neointima is constituted by proliferating smooth muscle cells originating from the media and extracellular matrix components secreted from these cells. This hyperplasic response is part of the wound-healing reaction. The angioplasty intervention causes a denudation of the intimal monolayer of endothelial cells. The denuded surface is highly thrombogenic and stimulates the adhesion and aggregation of blood platelets, that upon activation secrete several mitogens and cytokines (George, 2000). Moreover, the inflation of the balloon causes a mechanical stretching of the vessel and rupture of the internal elastic lamina, exposing the media to circulating mitogens, triggering the migration and proliferation of smooth muscle cells. The capacity of these cells to migrate through the struts of the stent is a main cause of the intra-stent restenosis. To prevent this process, the development of stents covered with biocompatible polymers allowing the local liberation of pharmacological agents (Regar, *et al*., 2001). Among the most promising drugs are two antimitogenic compounds Paclitaxel (Taxol™) and Rapamycin (Sirolimus™) that are able to limit the intrastent restenosis (Degertekin, *et al*., 2002; Heldman, *et al*., 2001; Morice, *et al*., 2002) . However, it was shown that this antimitogenic treatment local inhibit the process of reendothelialization (Farb, *et al*., 2001), which is important since the intact endothelium represents the best protection towards subsequent thrombosis at the level of the stent. An improvement of a local treatment of the atherosclerotic plaque therefore requires a stimulation of the regeneration of an endothelium with properties as close as possible those of the normal endothelium. The reendothelialization is, indeed, an essential mechanism for the inhibition of the neo-intimal hyperplasia.

The angiogenic factors possess activities which stimulate the reendothelialization: The VEGF (vascular endothelial cell growth factor) is a growth factor produced by a large number of cell types (notably in response to a hypoxia) which stimulates the migration and the proliferation of endothelial cells. However, the VEGF increases the endothelial permeability as well as the deposit of lipids and therefore favors the progression of the atherosclerotic plaque (Celletti, *et al*., 2001), which limits the potential interest of this factor.

The fibroblast growth factors (FGFs) constitute a family of at least 22 homologous proteins (Nakatake, *et al*., 2001; Ornitz and Itoh, 2001). FGFs act on a variety of cells by stimulating mitogenesis and/or by inducing morphological changes and differentiation. FGF-2 or basic FGF, one of the first described members of the FGF family, is involved in developmental processes, wound healing, and angiogenesis as well as in tumor progression (Bikfalvi, *et al*., 1997; Mason, 1994; Yamaguchi and Rossant, 1995). Among these factors, the FGF-1 (or acidic FGF) but especially the FGF-2 (or basic FGF) are known for their activities on the cells of the vascular wall. The FGF-2 is, with the VEGF, the most powerful angiogenic factor. It stimulates tubulogenesis, that is, the formation of capillaries and stimulates the proliferation and the migration of endothelial but also smooth muscular cells. Five FGF-2 isoforms of 18, 22, 22.5, 24, and 34 kDa are synthesized through an alternative translational initiation process (Arnaud, *et al*., 1999; Florkiewicz and Sommer, 1989; Prats, *et al*., 1989). These isoforms differ only in their NH2 extremities, which confer a nuclear localization to the four high molecular mass (HMM) CUG-initiated forms while the smaller AUG-initiated protein of 18 kDa is predominantly cytoplasmic or excreted and stored in the extracellular matrix (Bugler, *et al*., 1991; Quarto, *et al*., 1991). These FGF-2s can exert their effects through different pathways. The nuclear HMM forms of FGF-2 act in an intracrine manner that can influence tumor progression and promote cell proliferation under low-serum conditions (Arese, *et al*., 1999; Bikfalvi, *et al*., 1995; Couderc, *et al*., 1991; Okada-Ban, *et al*., 1999). The AUG-initiated 18 kDa protein is responsible for the auto-/paracrine action of FGF-2 and stimulates cellular proliferation and migration via interaction with (Bikfalvi, *et al*., 1995) high-affinity transmembrane tyrosine kinase receptors (FGFR) and low-affinity receptors (heparan sulphate-containing proteoglycans) (Johnson and Williams, 1993). Ligand activation of the FGFR causes autophosphorylation of the receptor and the activation of intracellular signaling pathways, in which mitogen-activated protein kinases (MAPK) and/or phospholipase C are important mediators (Cross and Claesson-Welsh, 2001; Friesel and Maciag, 1995). FGF-2 can internalize with both kinds of receptors into the cytoplasm (Gleizes, *et al*., 1995; Reiland and Rapraeger, 1993; Roghani and Moscatelli, 1992) and translocate into the nucleus during the G1 phase of the cell cycle (Baldin, *et al*., 1990) by an unknown mechanism distinct from that of HMM FGF-2 (Patry, *et al*., 1994).

Over the past ten years, accumulating data have suggested that, surprisingly, nuclear targeting and action of growth factors and growth factor receptors constitute a complementary signaling pathway involved in the induction of cell proliferation. In different target cells, nuclear association has been demonstrated for a large number of secreted growth factors or hormones such as FGF, Epidermal Growth Factor (EGF), Platelet Derived Growth Factor (PDGF), Nerve Growth Factor (NGF) and insulin (Jans, 1994; Keresztes and Boonstra, 1999; Mason, 1994; Stachowiak, *et al*., 1997). Several findings indicate that the nuclear translocation of extracellularly acting FGFs may also be required for the mitogenic effect in different cell types (Imamura, *et al*., 1990; Joy, *et al*., 1997; Wiedlocha, *et al*., 1994). Even though FIBP (Kolpakova, *et al*., 1998) and Casein Kinase 2 (Bailly, *et al*., 2000; Skjerpen, *et al*., 2002) could be mediators of these signals of FGF-1 and/or FGF-2, the details regarding the molecular mechanisms by which the FGFs exert their intracellular effects remain to be elucidated.

The three HMM and the 18 kDa intracellular FGFs are found as components of large protein complexes of 320 and 130 kDa, respectively (Patry, *et al*., 1997).

In an attempt to identify proteins associated to such complexes, a human placenta cDNA library was screened in the two-hybrid system. A previously unknown protein, named Translokin by the inventors, was characterized which is able to bind 18 kDa FGF-2 in a specific and direct way.

In the frame of the present invention, the inventors give the demonstration that Translokin co-immunolocalizes with β-tubulin, and by using an FGF-2/FGF-1 chimeric strategy and the inhibition of Translokin expression with a siRNA duplex, that Translokin mediates the translocation of FGF-2 to the nucleus and/or its vicinity.

The present invention relies mainly on the demonstration made by the inventors that FGF-2 derived proteins which are unable to interact specifically with Translokin, are defective in stimulating the proliferation of endothelial cells, but their tubulogenesis activity is preserved.

Thus, the main aim of the present invention is to provide new proteins which can be coated to medical devices, such as endovascular prostheses (stents), in order to promote the endothelialization of said medical devices and the reendothelialization of the vessel in the vicinity of the stent by preserving the tubulogenic and migrating capacity of endothelial cells without stimulating their proliferation.

The present invention relates to the use of animal or human basic fibroblast growth factor (FGF-2) derived proteins for the preparation of biomaterials or medical devices chosen among endovascular prostheses, such as stents and bypass grafts, or coated endoprostheses, or other kinds of medical prostheses, said FGF-2 derived proteins being chosen among FGF-2 mutants which are unable to interact specifically with the Translokin represented by SEQ ID NO: 2, i.e. having a binding capacity to said Translokin of less than 40 %, and more preferably of less than 10%, said FGF-2 mutants being such that they are defective in intracellular trafficking, and in stimulating the proliferation of endothelial cells, and that their tubulogenesis activity is preserved.

The binding capacity to said Translokin mentioned above, and which in the case of the FGF-2 mutants defined above is less than 40 %, and more preferably less than 10 %, is measured with comparison to bovine FGF-2 in the yeast two-hybrid system as detailed in the material and methods section hereafter.

By the expression "defective in intracellular trafficking" mentioned above, it should be understood that FGF-2 mutants according to the invention are unable to translocate from the cell surface to the nuclear compartment. Such property can be measured by subcellular fractionation of fibroblasts (such as NIH 3T3) or endothelial cells (such as ABAE) treated with HA-tagged FGF-2 mutants. Such property can also be assessed by immunocytochemistry of said cells. Detailed examples of these techniques are given in the material and methods section below.

The defective in stimulating the proliferation of endothelial cells property of the FGF-2 mutants mentioned above, can be evaluated by methods measuring cell proliferation, such as cell counting, incorporation of radioactive [H³]thymidine, MTT assay or other techniques used to assess cell proliferation, of cells treated with FGF-2 mutants. A detailed example of such a technique is given in the material and methods section below.

The tubulogenesis activity of the FGF-2 mutants mentioned above, can be evaluated by methods measuring the capacity of endothelial cells to form capillary structures. One such method is described in the the material and methods section below.

FGF-2 mutants according to the invention are able to stimulate the endothelialization of said medical devices, or the reendothelialization of a fully or partially deendothelialized blood vessel, said FGF-2 mutants being able to diminish the frequency of restenosis intra or extrastent.

Furthermore, FGF-2 mutants according to the invention are able to regenerate an endothelium with functional or morphological characteristics close to a normal endothelium. Such characteristiscs could be the capacity to produce NO, as described in (Darblade, *et al*., 2002). The morphology of the endothelium could be assessed by silver nitrate staining as descibed in the material and methods section below.

The invention relates more particularly to the use as defined above, of FGF-2 mutants corresponding to bovine FGF-2 represented by SEQ ID NO: 4, or human FGF-2 represented by SEQ ID NO: 6, or corresponding to any other animal FGF-2 showing 83 to 99 % of identical amino acids with SEQ ID NO: 4 or SEQ ID NO: 6, and wherein the region delimited by the amino acids located from positions 40 to 130 contains at least one mutation by deletion, or substitution by a heterologous amino acid natural or not, of at least one amino acid of said region, or by insertion of at least one heterologous amino acid natural or not in said region.

The invention concerns more particularly the use as defined above, of FGF-2 mutants corresponding to FGF-2 represented by SEQ ID NO: 4 or by SEQ ID NO: 6, wherein the region delimited by the amino acids located from positions 40 to 75, and/or the region delimited by the amino acids located from positions 105 to 130, contain at least one mutation as defined above.

The invention also concerns the use as defined above, of FGF-2 mutants corresponding to FGF-2 represented by SEQ ID NO: 4 or by SEQ ID NO: 6, wherein the region delimited by the amino acids located from positions 44 to 61 contains at least one mutation as defined above.

In this respect, the invention relates to the use as defined above, of FGF-2 mutants corresponding to FGF-2 represented by SEQ ID NO: 4 or by SEQ ID NO: 6, wherein at least one of the amino acids located in positions 44, 48, 52, 54, 55, 58, 61, 65, 68, 69, 71, and 73, is mutated.

The invention relates more particularly to the use as defined above, of FGF-2 mutants corresponding to FGF-2 represented by SEQ ID NO: 4 or by SEQ ID NO: 6, and comprising at least one of the following mutations : H44L, R48T, V52T, E54D, K55R, P58Q, K61Q, Q65S, E68S, R69V, V71E, and S73Y, said mutants being represented by SEQ ID NO: 8, or by SEQ ID NO: 20, respectively.

The invention also relates more particularly to the use as defined above, of the following FGF-2 mutants :
· the FGF-2 mutant comprising the mutations R48T, V52T, and E54D,
· the FGF-2 mutant comprising the mutations H44L, R48T, V52T, and K55R,
· the FGF-2 mutant comprising the mutations H44L, R48T, V52T, and E54D,
· the FGF-2 mutant comprising the mutations Q65S, E68S, R69V, V71E, and S73Y.

The invention concerns more particularly the use as defined above, of FGF-2 mutants corresponding to FGF-2 represented by SEQ ID NO: 4 or by SEQ ID NO: 6, wherein at least the amino acids in positions 54 and 55 are mutated, such as FGF-2 mutants comprising the mutations E54D and K55R.

In this respect, the invention relates more particularly to the use as defined above, of FGF-2 mutants corresponding to FGF-2 represented by SEQ ID NO: 4 or by SEQ ID NO: 6, wherein at least the amino acids in positions 54 and 55 are mutated, and wherein at least one of the amino acids located in positions 44, 48, 52, 58, and 61, is mutated, such as FGF-2 mutants comprising the mutations E54D and K55R, and at least one of the following mutations H44L, R48T, V52T, P58Q, and K61Q, and more particularly :
· the FGF-2 mutant comprising the mutations E54D, K55R, and H44L,
· the FGF-2 mutant comprising the mutations E54D, K55R, H44L, and R48T,
· the FGF-2 mutant comprising the mutations E54D, K55R, H44L, R48T, and V52T,
· the FGF-2 mutant comprising the mutations E54D, K55R, H44L, R48T, V52T, P58Q, and K61Q.

The invention more particularly concerns the use as defined above, of FGF-2 mutants corresponding to chimeric FGF-1/FGF-2 proteins.

In this respect, the invention concerns the use as defined above, of the following FGF-2 mutants :
- Nb1a2, represented by SEQ ID NO: 10, corresponding to a chimeric FGF-1/FGF-2 protein wherein the amino acids in positions 1 to 43 and 79 to 155 belong to bovine FGF-2 represented by SEQ ID NO: 4, and the amino acids located in positions 44 to 78 belong to human FGF-1 represented by SEQ ID NO: 14,
- Nb1a1.6, represented by SEQ ID NO: 12, corresponding to a chimeric FGF-1/FGF-2 protein wherein the amino acids in positions 1 to 43 belong to bovine FGF-2 represented by SEQ ID NO: 4, the amino acids located in positions 44 to 61 belong to human FGF-1 represented by SEQ ID NO: 14, and the amino acids located in positions 62 to 155 belong to human FGF-2 represented by SEQ ID NO: 6.

The invention also concerns the use as mentioned above, of FGF-2 mutants corresponding to FGF-2 represented by SEQ ID NO: 4 or by SEQ ID NO: 6, wherein the region delimited by the amino acids located from positions 109 to 126 contains at least one mutation as defined above.

The invention concerns more particularly the use as defined above, of FGF-2 mutants corresponding to FGF-2 represented by SEQ ID NO: 4 or by SEQ ID NO: 6, wherein at least one of the amino acids located in positions 109, 111, 116, 118, 120, 121, 122, 124, and 126, is mutated.

In this respect, the invention concerns more particularly the use as defined above, of FGF-2 mutants corresponding to FGF-2 represented by SEQ ID NO: 4, or by SEQ ID NO: 6, and comprising at least one of the following mutations : S109E, N111H, R116T, R118K, Y120H/A/E, S/T121A/E/K, S122E/K/N, Y124F, and A126G, said mutants being represented by SEQ ID NO: 16, or by SEQ ID NO: 22, respectively.

The invention relates more particularly to the use as defined above, of FGF-2 mutants corresponding to FGF-2 represented by SEQ ID NO: 4, or by SEQ ID NO: 6, and comprising the following mutations : S109E, N111H, Y120H/A/E, S/T121A/E/K, S122E/K/N, Y124F, and A126G.

The invention concerns more particularly the use as defined above, of the FGF-2 mutant represented by SEQ ID NO: 18, corresponding to a chimeric FGF-1/FGF-2 protein wherein the amino acids in positions 1 to 108 and 127 to 155 belong to human FGF-2 represented by SEQ ID NO: 6, and the amino acids located in positions 109 to 126 belong to human FGF-1 represented by SEQ ID NO: 14.

The invention also concerns the FGF-2 derived proteins chosen among the following FGF-2 mutants :
- FGF-2 mutants corresponding to FGF-2 represented by SEQ ID NO: 4, or by SEQ ID NO: 6, and comprising at least one of the following mutations : H44L, R48T, V52T, E54D, K55R, P58Q, K61Q, Q65S, E68S, R69V, V71E, and S73Y, said mutants being represented by SEQ ID NO: 8, or by SEQ ID NO: 20, respectively, and more particularly:
   · the FGF-2 mutant comprising the mutations R48T, V52T, and E54D,
   · the FGF-2 mutant comprising the mutations H44L, R48T, V52T, and K55R,
   · the FGF-2 mutant comprising the mutations H44L, R48T, V52T, and E54D,
   · the FGF-2 mutant comprising the mutations Q65S, E68S, R69V, V71E, and S73Y,
- FGF-2 mutants corresponding to FGF-2 represented by SEQ ID NO: 4, or by SEQ ID NO: 6, wherein at least the amino acids in positions 54 and 55 are mutated, such as FGF-2 mutants comprising the mutations E54D and K55R,
- FGF-2 mutants corresponding to FGF-2 represented by SEQ ID NO: 4, or by SEQ ID NO: 6, wherein at least the amino acids in positions 54 and 55 are mutated, and wherein at least one of the amino acids located in positions 44, 48, 52, 58, and 61, is mutated, such as FGF-2 mutants comprising the mutations E54D and K55R, and at least one of the following mutations H44L, R48T, V52T, P58Q, and K61Q, and more particularly :
   · the FGF-2 mutant comprising the mutations E54D, K55R, and H44L,
   · the FGF-2 mutant comprising the mutations E54D, K55R, H44L, and R48T,
   · the FGF-2 mutant comprising the mutations E54D, K55R, H44L, R48T, and V52T,
   · the FGF-2 mutant comprising the mutations E54D, K55R, H44L, R48T, V52T, P58Q, and K61Q
- Nbla2, represented by SEQ ID NO: 10, corresponding to a chimeric FGF-1/FGF-2 protein wherein the amino acids in positions 1 to 43 and 79 to 155 belong to bovine FGF-2 represented by SEQ ID NO: 4, and the amino acids located in positions 44 to 78 belong to human FGF-1 represented by SEQ ID NO: 14,
- Nbla1.6, represented by SEQ ID NO: 12, corresponding to a chimeric FGF-1/FGF-2 protein wherein the amino acids in positions 1 to 43 belong to bovine FGF-2 represented by SEQ ID NO: 4, the amino acids located in positions 44 to 61 belong to human FGF-1 represented by SEQ ID NO: 14, and the amino acids located in positions 62 to 155 belong to human FGF-2 represented by SEQ ID NO: 6,
- FGF-2 mutants corresponding to FGF-2 represented by SEQ ID NO: 4, or by SEQ ID NO: 6, and comprising at least one of the following mutations : S109E, N111H, R116T, R118K, Y120H/A/E, S/T121A/E/K, S122E/K/N, Y124F, and A126G, said mutants being represented by SEQ ID NO: 16, or by SEQ ID NO: 22, respectively,
- FGF-2 mutants corresponding to FGF-2 represented by SEQ ID NO: 4, or by SEQ ID NO: 6, and comprising the following mutations : S109E, N111H, Y120H/A/E, S/T121A/E/K, S122E/K/N, Y124F, and A126G,
- the FGF-2 mutant represented by SEQ ID NO: 18, corresponding to a chimeric FGF-1/FGF-2 protein wherein the amino acids in positions 1 to 108 and 127 to 155 belong to human FGF-2 represented by SEQ ID NO: 6, and the amino acids located in positions 109 to 126 belong to human FGF-1 represented by SEQ ID NO: 14.

The invention also concerns the nucleotide sequences encoding FGF-2 derived proteins as defined above.

The invention also relates to the vectors, such as plasmids, containing the nucleotide sequences as mentioned above.

The invention also concerns the host cells, such as *Escherichia coli* or other unicellular organisms, transformed with vectors as defined above.

The invention also relates to a process for the preparation of FGF-2 derived proteins as defined above, comprising the culturing of host cells in an appropriate medium, and the purification of said FGF-2 derived proteins produced.

The invention also relates to biomaterials or medical devices coated with animal or human basic fibroblast growth factor (FGF-2) derived proteins such as defined above.

The invention relates more particularly to biomaterials or medical devices as mentioned above, coated with basic fibroblast growth factor (FGF-2) derived proteins as defined above, said biomaterials or medical devices being chosen among endovascular prostheses, such as stents used for inhibiting restenosis or stenosis following angioplasty.

For illustration purpose, such endovascular prostheses and methods for coating proteins thereto, are more particularly described in WO 9749434, or are those currently used in the art. The compounds used for the coating of the prostheses should preferentially permit a controlled release of the FGF-2 derived proteins. Said compounds could be polymers (such as sutures, polycarbonate, Hydron, and Elvax), biopolymers/biomatrices (such as alginate, fucans, collagen-based matrices, heparan sulfate) or synthetic compounds such as synthetic heparan sulfate-like molecules or combinations thereof (Davies, *et al*., 1997; Desgranges, *et al*., 2001; Dixit, *et al*., 2001; Ishihara, *et al*., 2001; Letourneur, *et al*., 2002; Tanihara, *et al*., 2001; Tassiopoulos and Greisler, 2000).

The invention also relates to a process for the screening of FGF-2 mutants according to the invention, said process comprising :
- measurement of the binding capacity of said FGF-2 mutants to Translokin mentioned above, for example in a two-hybrid system as detailed hereafter, and selection of those mutants having less than 40%, and more preferably less than 10%, of binding capacity to Translokin when compared to bovine FGF-2,
- measurement of the property of the FGF-2 mutants selected in the previous step of being unable to translocate from the cell surface to the nuclear compartment ; such property could be measured by subcellular fractionation of fibroblasts (such as NIH 3T3) or endothelial cells (such as ABAE) treated with HA-tagged FGF-2 mutants ; this could also be done using other cell types for which FGF-2 is a mitogen. Such property could also be assessed by immunocytochemistry of said cells. Detailed examples of these techniques are given in the material and methods section below,
- measurement of the property of the FGF-2 mutants selected in the previous steps of being defective in stimulating the proliferation of endothelial cells ; such property could be evaluated by methods measuring cell proliferation (such as cell counting, incorporation of radioactive [H³]thymidine, MTT assay or other techniques used to assess cell proliferation) of cells treated with FGF-2 derived mutants ; a detailed example of such a technique is given in the material and methods section below,

The tubulogenesis activity of the FGF-2 mutants mentioned above, could be evaluated by methods measuring the capacity of endothelial cells to form capillary structures. One such method is described in the the material and methods section below.

The invention is further illustrated with the detailed description which follows of mutants according to the present invention, and their biological properties.

### RESULTS

### Isolation of a novel FGF-2-interacting protein

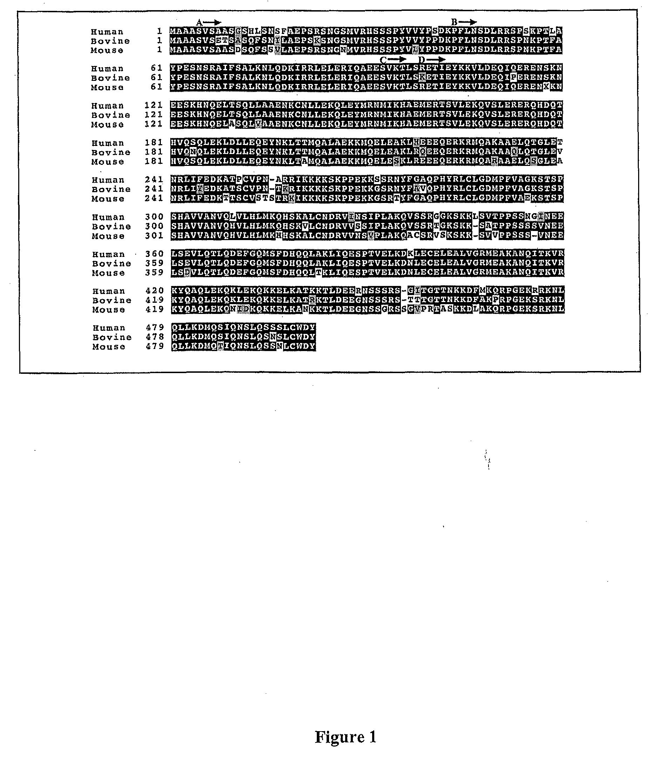

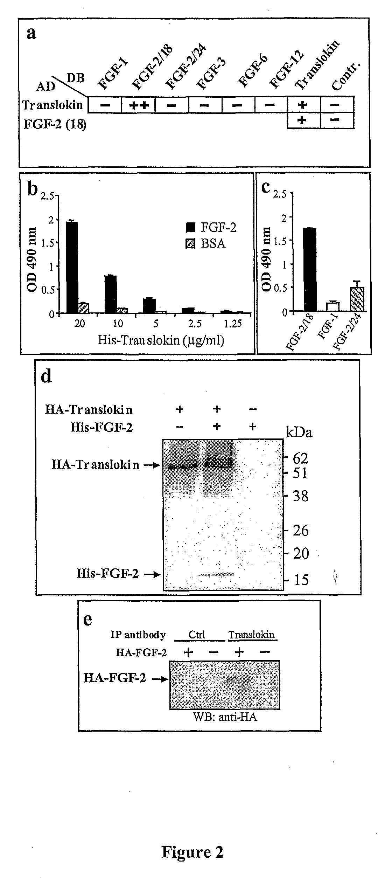

A yeast two-hybrid method was used to identify intracellular proteins that interact with FGF-2. Using the 18 kDa FGF-2 isoform as bait we screened a human placental complementary DNA library (Clontech). Four positive and distinct clones similar to the KIAA0092 mRNA (Nagase, *et al*., 1995) were isolated, all encoding the major part of a previously non-characterized protein (Fig. 1). After a more substantial characterization of its function (see below), the protein was named Translokin. The full-length cDNAs encoding human, mouse and bovine Translokin were amplified, cloned and sequenced using either Genebank (human) or EST data (mouse, bovine). The open reading frames encode 500, 500 and 499 amino acids respectively (Fig. 1). The three predicted proteins share an overall sequence identity of 88%, indicating a high degree of evolutionary conservation. To ascertain the specificity of the two-hybrid assay for the observed interaction between Translokin and the 18 kDa FGF-2, Translokin was coexpressed in yeast with a panel of representative FGF-family members (FGF-1, FGF-3, FGF-6, FGF-12), as well as the larger 24 kDa FGF-2. As shown in figure 2a, among the FGFs tested, Translokin was only able to interact with the 18 kDa FGF-2. Surprisingly, Translokin did not interact with the 24 kDa FGF-2, suggesting that the N-terminal extension of this FGF-2 isoform creates a sterical obstacle blocking the interaction at least in its fusion state in yeast. aA homotypic interaction was detected between Translokin molecules indicating its ability to form homodimers. The strength of the Translokin/FGF-2 interaction is noticeable since Translokin interacted with FGF-2 with similar efficiency as the positive control provided by the manufacturer (SV-40 large-T antigen interaction with p53). (Van den Berghe, *et al*., 2000). To ascertain that the Translokin/FGF-2 interaction was direct and independent of a third partner, the two proteins were expressed in *E.coli* and purified. The interaction between the purified proteins was tested in an *in vitro* ELISA assay. As shown in figure 2b, Translokin and FGF-2 interact strongly in a direct and dose-dependent manner. This assay was also used to verify *in vitro* the results obtained in the two-hybrid assay concerning the specificity of the interaction between 18 kDa FGF-2 and Translokin. For this, the interaction between Translokin and FGF-1 or 24 kDa FGF-2 was tested, since these two proteins are the most closely related to 18 kDa FGF-2. The lack of interaction between Translokin and FGF-1 was confirmed (figure 2c). However, the interaction obtained between Translokin and 24 kDa FGF-2 was slightly greater than the one obtained in yeast, but remained low compared to the 18 kDa FGF-2/Translokin interaction. To further study the FGF-2/Translokin interaction, coimmunoprecipitation experiments were performed on NIH-3T3 cells, which are known to internalize exogenously added FGF-2 (Bailly, *et al*., 2000). NIH-3T3 cells, transfected with a plasmid encoding haemagglutinin A (HA)-tagged Translokin, were incubated with or without His-tagged FGF-2 (18 kDa). Immunoprecipitation of Translokin, with an anti-HA antibody, co-precipitated His-FGF-2 as detected by Western-blot analysis of the precipitates with an anti-His antibody (Fig. 2d). The same results were obtained by immunoprecipitation of endogenous Translokin. For this, lysates of NIH-3T3 cells stimulated or not with HA-tagged FGF-2, were incubated with anti-Translokin or with a control antibody. As shown figure 2e, anti-Translokin was able to specifically co-immunoprecipitate the HA-FGF-2. Taken together, these data show that 18 kDa FGF-2 and Translokin are able to interact *in vivo* and *in vitro.*

### Expression and subcellular localization of Translokin

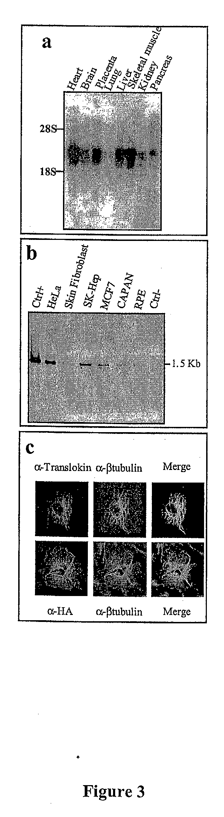

The distribution of Translokin mRNA expression in human tissues was next examined by hybridizing a multi-tissue poly(A) Northern blot (Clontech) with a human Translokin cDNA probe. The expression was widespread with highest mRNA levels in heart and skeletal muscle and lowest in lung. A major 3.2 kilobase messenger-RNA species was detected in all human tissues tested as well as two other transcripts of approximately 2.6 and 2.2 kb (Fig. 3a). In accordance with these data, a 1500 bp fragment corresponding to a unique ORF in all human cell lines examined has been detected by RT-PCR (Fig. 3b). A polyclonal antibody against Translokin, developed in the laboratory, detected a protein in all cell lines tested of human, simian, murine and bovine origin, with a molecular mass of 55 kDa corresponding well with the predicted one. Taken together, these results reflect a ubiquitous expression of Translokin. The subcellular localization of Translokin was investigated in COS-7 cells, transfected with a plasmid encoding HA-tagged Translokin. The monoclonal α-HA or the polyclonal α-Translokin antibodies revealed the same cytoplasmic distribution that entirely overlapped with the staining of microtubules obtained with an anti-β-tubulin antibody (Fig. 3c) and confirmed by the bright color in the merged pictures.

### The Translokin-FGF-2 interaction involves two distinct regions in FGF-2

The inventors subsequently wanted to determine the structural elements in FGF-2 involved in the interaction between FGF-2 and Translokin, in the aim of generating a non-interacting FGF-2 mutant, which would serve as a tool for the evaluation of the physiological role of the Translokin/FGF-2 interaction.

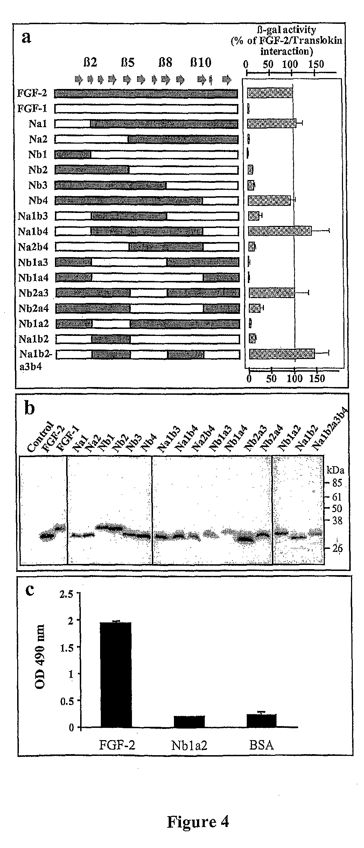

The chosen strategy was based on the fact that Translokin is unable to interact with FGF-1, despite the high degree of sequence identity (53%) shared by FGF-1 and 2 and the close resemblance of their three-dimensional structures (Zhu, *et al*., 1991). This prompted the inventors to choose a cassette-shuffling approach, thereby paying respect to the preservation of the overall structure of FGF. The chimeric FGF-1/FGF-2-proteins were fused with GAL4 DNA-binding domain and the ability of the fusion proteins to interact *in vivo* with Translokin was examined in the yeast two-hybrid system (Fig. 4a). The rationale of the nomenclature of the chimeric proteins is explained in the legend of figure 4. The expression level of the chimeric proteins was assessed by Western blotting using an anti-GAL4DBD monoclonal antibody (Fig. 4b). The results demonstrate the absolute requirement of two separate regions in order to maintain the interaction. The first region is located between β-sheets 2 and 5 (compare Na1 with Na2 or Nalb4 with Na2b4). The replacement in FGF-2 of the equivalent amino acids from FGF-1 (Nb1a2) and the reverse chimera (Nalb2) demonstrated respectively the indispensability but also the insufficiency of this region. Indeed, a second region positioned between β-sheets 8 and 10 is important in order to maintain the interaction (compare Nb2a4 with Nb2a3, Nb3 with Nb4 or Nalb3 with Na1b4). The amino acids comprised within these two regions were therefore replaced in FGF-1, by the corresponding FGF-2 sequences (amino acids 44-78 and 109-126). The fact that this chimera (Nalb2a3b4) exhibited the same capacity to interact with Translokin as FGF-2, demonstrate that the structural motifs involved in the interaction with Translokin are present within these two regions in FGF-2. In the non-interacting FGF-2 mutant, Nb1a2, the FGF-1-derived sequence is restricted to 12 amino acids in the first region. This chimera was chosen to confirm *in vitro* the results obtained in the *in vivo* two-hybrid system. For this, recombinant Nb1a2 was produced in *E. coli* and purified by heparin sepharose chromatography. The ability of the recombinant Nb1a2 protein to interact with Translokin *in vitro* was then tested in an ELISA-based assay. As shown in figure 4c, Nb1a2 has no affinity for Translokin.

### Characterization of Nb1a2

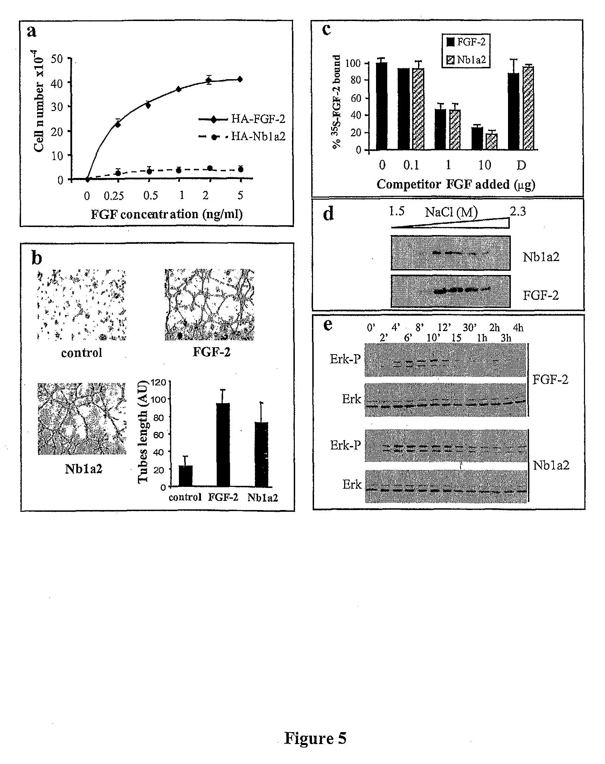

FGF-2 is a potent mitogen for endothelial cells. Proliferation assays were thus performed with Adult Bovine Aortic Endothelial cells (ABAE), by adding different doses of recombinant FGF-2 or Nb1a2 in culture media. Compared to FGF-2, Nb1a2 exhibited a very low mitogenic activity. The maximal mitogenic effect was obtained for 2 ng/ml of FGF-2 (Fig. 5a), whereas the mitogenic potential of Nb1a2 at this concentration was reduced by 90%. In stark contrast, Nb1a2 was equally efficient as FGF-2 in stimulating tube formation of endothelial cells in Matrigel™ (Fig. 5b) as well as and cell migration.

These results demonstrate that the mitogenic and differentiation activities of FGF-2 can be uncoupled by mutations affecting the FGF-2/Translokin interaction.

FGF-mediated activities require the binding of the growth factor to its high-affinity tyrosine kinase receptors. The binding of FGF-2 and Nb1a2 to the FGF receptors was therefore examined by comparing the ability of increasing concentrations of FGF-2 and Nb1a2 to displace [³⁵S]-labeled FGF-2, bound to ABAE cell surface receptors. As shown in figure 5c, both FGF-2 and Nb1a2 displaced the binding of FGF-2 with the same efficiency, indicating equivalent affinities for the high-affinity binding sites on the cell surface. Heparan sulphate-containing proteoglycans (HSPG) are also implicated in FGF-2 mediated signalization, representing low-affinity binding sites. The competition experiments for these sites showed similar results, which is in agreement with the affinity profile towards heparin-sepharose chromatography (Fig. 5d). Moreover, it is well known that phosphorylation and activation of mitogen-activated protein kinases Erk1 and Erk2 are required for the mitogenic response induced by FGF-2 (Cross and Claesson-Welsh, 2001; Pages, *et al*., 1993). By immunoblotting experiments with antibodies raised against phosphorylated forms of Erk1 and 2, it has been observed that FGF-2 and Nb1a2 were both able to activate p42 and p44 in ABAE cells for comparable time lengths (Fig. 5e). Between 4 and 12 hours, the Erk-phosphorylation was low, similar and decreased with the same kinetics in both cases. Activation of p70S6 kinase (a serine/threonine kinase that phosphorylates the ribosomal protein S6 in response to a number of extracellular stimuli) is required for FGF-stimulated cell proliferation but not differentiation of endothelial cells (Kanda, *et al*., 1997). As for Erk1/2 no difference between FGF-2 and Nb1a2 was found when the phosphorylation status of p70S6K by immunoblotting was assessed. These results show that Nb1a2 is able to activate the main signaling pathways involved in the mitogenic response induced by FGF-2.

### Internalized FGF-2 but not Nb1a2 is associated with the nucleus

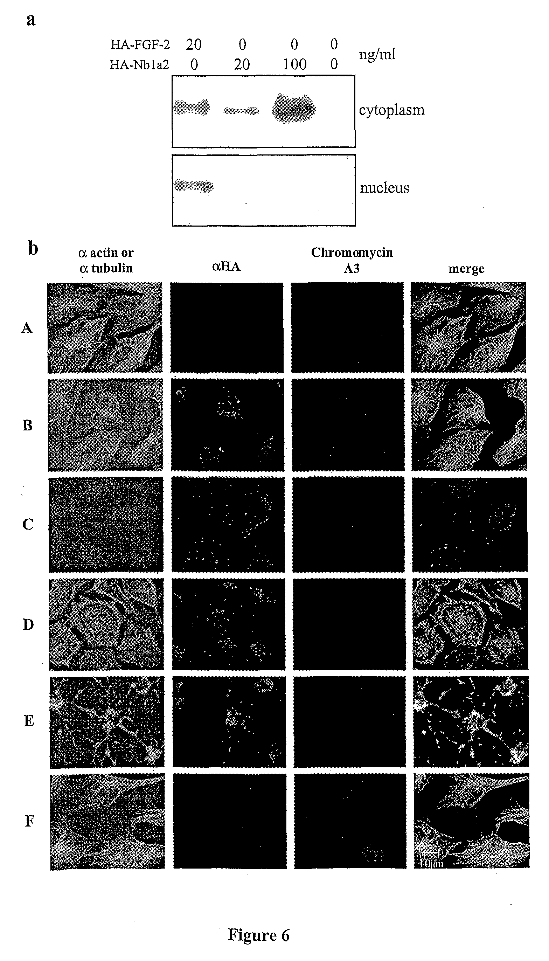

Exogenously added FGF-2 translocates to the nucleus of proliferating cells (Baldin, *et al*., 1990; Bouche, *et al*., 1987; Clarke, *et al*., 2001; Hawker and Granger, 1992; Reilly and Maher, 2001; Walicke and Baird, 1991) and this nuclear translocation is associated with a proliferative state of the treated cells (Baldin, *et al*., 1990; Joy, *et al*., 1997). The inventors therefore tested whether exogenously added Nb1a2 retained the ability of FGF-2 to be internalized from the outside of the cell to the cytoplasm and further translocated to the nucleus. For this purpose, 50% confluent ABAE cells, rendered quiescent after 48h under serum-free conditions, were incubated for 2 h with FGF-2 or Nb1a2. To discriminate from endogenous and exogenous FGF-2, HA-tagged proteins were used. After cell harvesting and fractionation, the respective amounts of HA-FGF-2 or HA-Nb1a2 in the cytoplasmic and nuclear fractions were determined by immunoblotting with an α-HA antibody. As shown in figure 6a, both HA-FGF-2 and HA-Nb1a2 were detected in the cytoplasmic fraction. However, only the HA-FGF-2 was found in the nuclear fraction. These results show that mutations disturbing the interaction between FGF-2 and Translokin lead to a loss of mitogenicity. This could be explained by the inability of the mutant Nb1a2 to be translocated to the nucleus and/or its vicinity. To verify these results, immunofluorescence studies and confocal analysis of the intracellular trafficking of exogenous FGF-2 were performed.

The data, presented in figure 6b (panels B, D), show the distribution of internalized HA-FGF-2 in ABAE cells after two hours incubation at 37°C. The growth factor appeared as intracellular dots, indicating uptake in vesicles, with a prominent perinuclear FGF-2 staining and a relatively sparse FGF-2 staining within the nucleus. The staining obtained with α-HA was specific as no staining was observed when the growth factor was omitted (Fig. 6b panel A). In agreement with the data obtained by cell fractionation (Fig. 6a), no nuclear-associated staining was observed when cells were incubated with HA-Nb1a2 (Fig. 6b panel F). A double-labeling experiment with anti-β-tubulin, resulted in a good overlap of β-tubulin and HA-FGF-2, concentrated in the centrosome area. To verify if the intracellular transport of exogenous FGF-2 was dependent on microtubules, experiments with depolymerizing drugs were carried out. As shown in figure 6b (E), cytochalasin B, which induces depolymerization of actin microfilaments (compare the actin filament distribution in panels D and E), did not alter the distribution of the growth factor in the nuclear region. On the contrary, treatment with nocodazole, which causes depolymerization of microtubules (compare the microtubule organization in panel B with the one in panel E), prevented the appearance of the growth factor in the juxtanuclear region (panel C). In these cells the punctuated FGF-2 staining was found in the cytoplasm, especially in the vicinity of the plasma membrane. These results strongly suggest that the intracellular trafficking of exogenous 18 kDa FGF-2 is mediated by microtubules in ABAE cells and correlates with the microtubule-associated pattern of Translokin observed by immunocytochemistry (Fig. 3c).

### Nuclear association of internalized FGF-2 is essential for its mitogenic activity and is mediated by Translokin

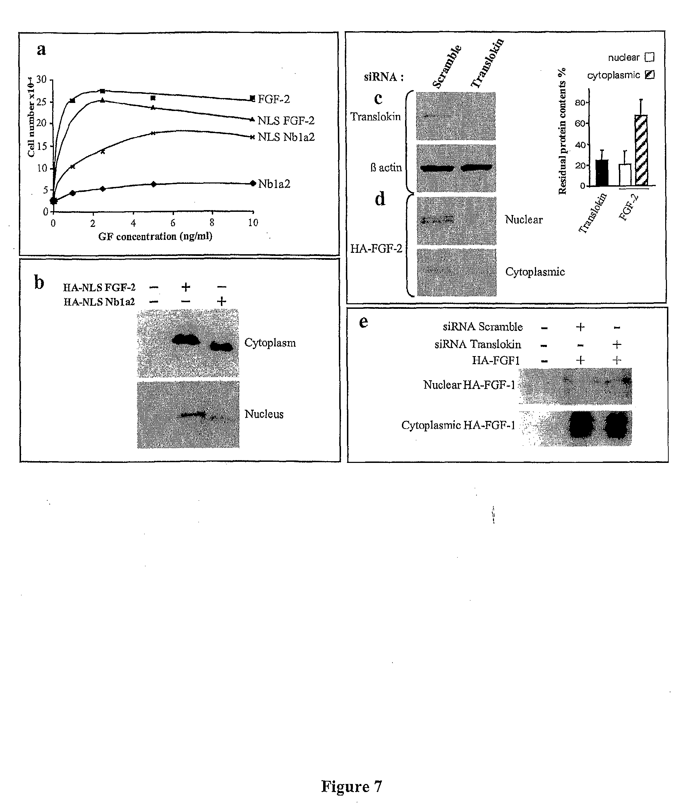

The inventors reasoned that if nuclear translocation was important for mitogenic activity of FGF-2, it may be possible to restore the mitogenic activity of Nb1a2 by the addition of a well characterized nuclear localization sequence (NLS). Therefore, chimeric polypeptides NLS Nb1a2 and NLS FGF as a control, containing the NLS derived from the SV-40 T antigen (PKKKRKKV) at the NH₂-terminus, were constructed. The chimeric proteins were produced in *Escherichia coli* and purified by heparin sepharose chromatography. Both proteins exhibited the same affinity as FGF-2 for immobilized heparin. They were then tested for their mitogenic activity in an *in vitro* proliferation assay using ABAE cells. As shown in figure 7a, the mitogenic activities of FGF-2 and NLS-FGF-2 were comparable. However, the addition of the NLS to Nb1a2 strongly enhanced its mitogenic activity, which reached approximately 60% of the one obtained with WT FGF-2. A fractionation experiment performed on NIH-3T3 cells stimulated or not with NLS-FGF-2 or NLS-Nb1a2, confirmed the ability of these growth factors to be translocated to the nuclear region (Fig. 7b). These results show that the nuclear association of FGF-2 is a critical step for its mitogenic activity.

To further investigate whether Translokin was the mediator for this translocation, NIH-3T3 cells were transfected with small interfering RNA (siRNA) duplexes (Elbashir, *et al*., 2001) to reduce the expression of endogenous Translokin. The inventors performed a single transfection with a siRNA targeting Translokin or with a scrambled siRNA as control. After 55h of transfection, cells were exposed to 20 ng/ml of HA-tagged FGF-2 for 2h. The cells were then collected and fractionated in order to study the respective amounts of FGF-2 in the cytoplasmic and nuclear fractions. Since the translocation of FGF-2 is dependent on the cell cycle and on the confluence state of the cells (Baldin, *et al*., 1990), great care was taken when seeding the cells. In accordance with the putative role of Translokin in FGF-2-induced proliferation, NIH-3T3 cells transfected with Translokin-targeted siRNA grew more slowly than cells transfected with control siRNA. These cells were therefore seeded at a slightly higher density in order to obtain exactly the same number of cells in both conditions at the time the HA-FGF-2 was added. After transfection the protein levels were evaluated by immunoblot analysis. As shown in figure 7c, the specific siRNA led to a decrease of about 78% of Translokin (average obtained from three independent experiments) whereas β-actin remained unaffected. Immunofluorescence studies showed that siRNA treatment did not affect the cytoskeletal organization. Interestingly, the decrease in Translokin expression by siRNA was correlated with a reduction of FGF-2 nuclear accumulation of about 80% (Fig. 7d). A diminution of the cytoplasmic HA-FGF-2 contents in cells transfected with the Translokin siRNA was also observed. However, this decrease, not exceeding 50%, was always less than the one observed for nuclear FGF-2 (Fig. 7d). This could reflect a participation of Translokin complex in the uptake or in the intracellular stability of exogenously added FGF-2. To assess the specificity of the Translokin-siRNA treatment on the FGF-2 translocation, the consequences of the decrease in Translokin expression, on FGF-1 trafficking, were tested. For this purpose, an HA-tagged FGF-1 has been produced and purified. The inventors then proceeded in the same way as for FGF-2. As shown in figure 7e, Translokin-siRNA treatment did not affect the translocation of HA-FGF-1. Taken together these results further demonstrate a direct role of Translokin in the translocation of FGF-2, a step that appears absolutely necessary for the FGF-2 mediated mitogenic activity.

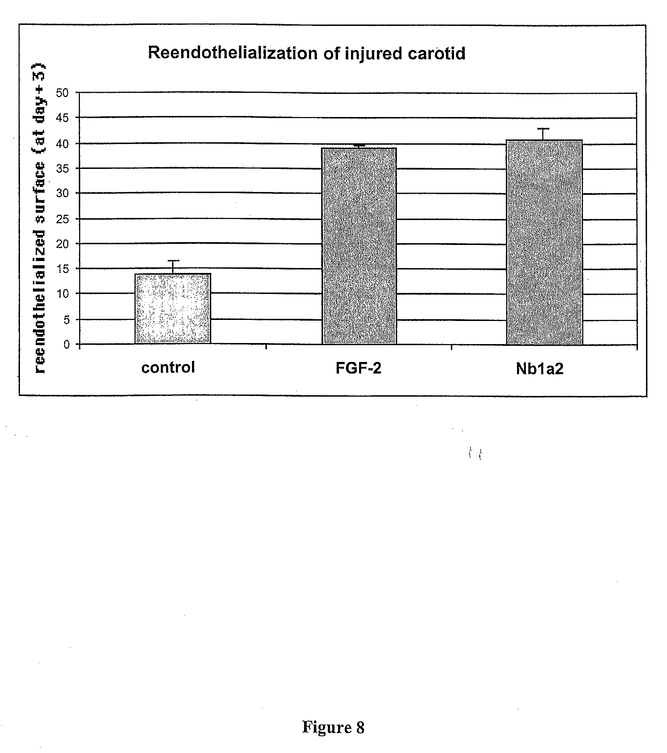

Nb1a2 accelerates the reendothelialisation of injured arteries FGF-2 has been shown to stimulate the regrowth of endothelium after balloon catheter denudation of the rat carotid artery (Lindner, *et al*., 1990), However, since FGF-2 is able to stimulate the proliferation and migration of smooth vascular cells (Jackson and Reidy, 1993; Lindner, *et al*., 1991) and thereby also increases and aggravates the neointima after injury (Lindner and Reidy, 1991), it does not constitute a suitable therapeutic tool for arterial healing. Given the characteristics of the biological activity of the Nb1a2 *in vitro,* as described in figure 5, the FGF-2 derived proteins with a strongly reduced binding capacity to Translokin but with maintained tubulogenic activities, could be more appropriate than wildtype FGF-2 as therapeutic agents to accelerate the arterial healing of injured arteries *in vivo*. To investigate this, an electric injury mouse model was used, which was developed by Carmeliet and coll. (Carmeliet, *et al*., 1997) and further adapted in the inventors' laboratory (Brouchet, *et al*., 2001). In this model, an electric pulse is applied to the distal part of the common carotid artery using a bipolar microregulator. Three days following injury, the endothelial regeneration process was evaluated by staining the denuded areas with Evans blue. As expected, 5 µg of FGF-2 (injected in bolus just after the injury) stimulated the endothelial regrowth (30% above saline-treated controls) (Fig.8). Interestingly, Nb1a2 was as efficient in accelerating the reendothelialisation of the artery.

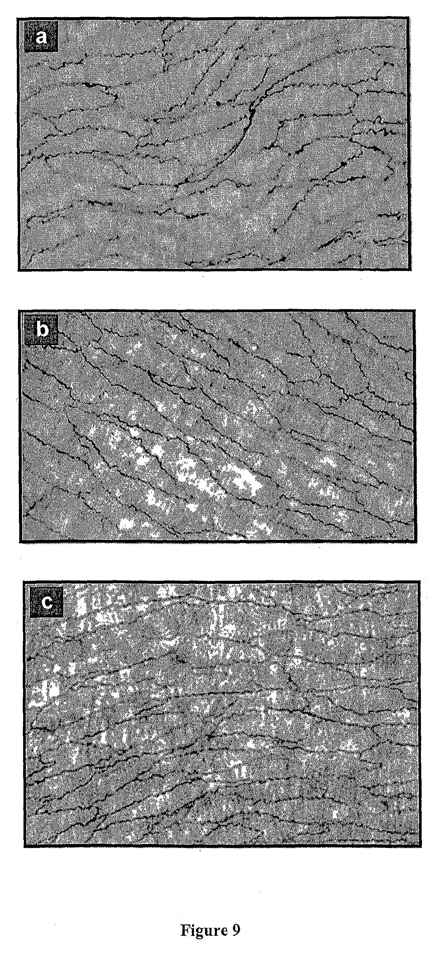

The formation of the neointima after vascular injury after treatment with FGF-2 or Nb1a2 was investigated in mice, sacrificed ten days after injury. Parafin embedded sections of fixed carotids, stained with hematoxylin, were examined microscopically. Whereas FGF-2 provoked a thickening of the intima (neointima) at the level of injury, no thickening of the intima was observed in mice treated with Nb1a2. The morphology of the regenerated endothelium was compared by silver nitrate staining of carotids from mice treated with FGF2 or Nb1a2, ten days after injury, as well as from non-injured control animals. As shown in figure 9b and 9c, the shape and the orientation of the endothelial cells of the carotids in the two latter groups were homogenous, following the longitudinal axis of the artery, whereas in the animals treated with FGF-2, the shape and the orientation of the cells were much less regular.

### Detailed analysis of the first Translokin-interacting region in FGF-2

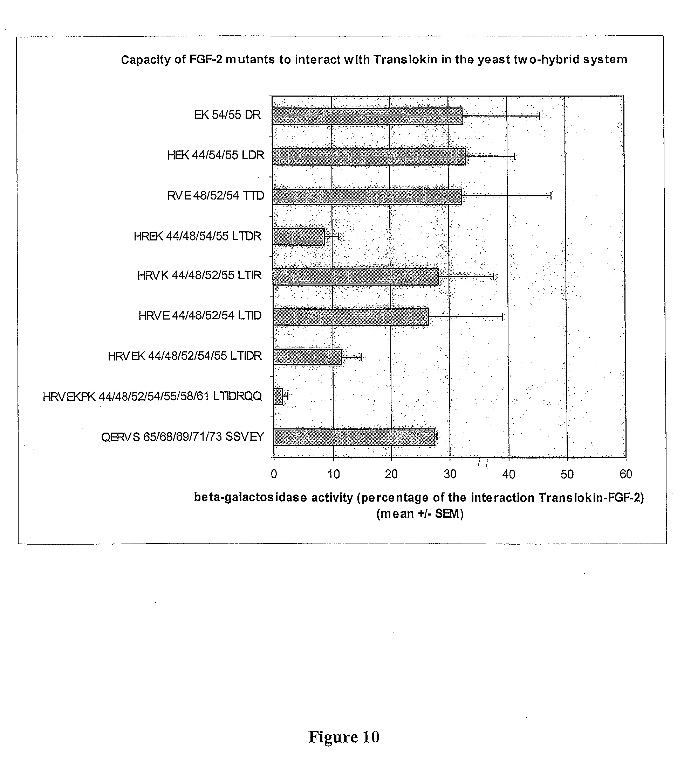

As described above, two regions in FGF-2, located between β-strands 2-5 and 8-10, respectively, are important for the interaction with Translokin. As shown in figure 4a, the structural entities within the first region constitute the most critical parameter for maintaining the interaction. In this region, 12 amino acids differ between human FGF-1 and human (or bovine) FGF-2. In order to more precisely define which ones of these amino acids that are implicated in the interaction, a number of more targeted FGF-2 mutants were generated. The capacity of these mutants to interact with Translokin was subsequently assessed in the yeast two-hybrid system. The data, represented in figure 10, show the necessity to mutate several amino acids in order to reduce the affinity to Translokin to a level comparable to the one of FGF-1 or Nb1a2. Indeed, only a hepta-amino acid mutant: HRVEKPK 44/48/52/54/55/58/61 LTIDRQQ (also referred to as Nb1a1.6) was completely void of any affinity for Translokin. In analogy with these results, similar studies of the second Translokin-interacting region in FGF-2, favors an importance of the entire region between β-strands 8 and 10, more than a role of the individual amino acids (data not shown).

### Discussion

Over the past decade, it has become increasingly apparent that a number of peptides hormones and growth factors elicit their biological responses in a bi-functional manner, both indirectly with cell surface receptors linked to conventional signal transduction pathways, as well as through direct association with the nuclei of target cells. This growing list of peptides includes fibroblast growth factors and in particularly FGF-2 and FGF-1, insulin, EGF, NGF, PDGF, prolactin, angiogenin and parathyroid hormone-related peptide (PTHrP) (Jans, 1994; Keresztes and Boonstra, 1999). Among these examples, FGF-1 and FGF-2 are prototypic for this dual pathway. For both factors the first and indispensable step is the cell surface recognition of the low and high affinity receptors. This step seems to be sufficient to exert the differentiation properties of the factor. Nevertheless, very little is known about intracellular routing of internalized FGFs, even though localization of FGF-2 has been reported in caveolae prior the endosome/lysosome pathway (Gleizes, *et al*., 1996). Internalization and nuclear translocation of FGF-2 have been abundantly reported and in several studies associated with cell proliferation (Bailly, *et al*., 2000; Baldin, *et al*., 1990; Joy, *et al*., 1997). Similar results have been reported for FGF-1 and different groups have clearly demonstrated that binding and activation of cell surface receptors are not solely sufficient for a full mitogenic response (Grieb and Burgess, 2000; Imamura, *et al*., 1990; Wiedlocha, *et al*., 1994), conferring an intracellular activity on internalized FGF. Some intracellular targets of FGF-2 have been previously described including Tax, FIF and CKII, (Bonnet, *et al*., 1996; Shen, *et al*., 1998; Van den Berghe, *et al*., 2000). CKII has also been recently described as an essential target of the intracellular FGF-1 (Skjerpen, *et al*., 2002). This interaction, as well as the MAPK pathway, are necessary for the proliferative effect of the factor. Furthermore, internalized FGFR1 has been found to be translocated to the nucleus in response to FGF-2 (Reilly and Maher, 2001). This could also constitute a key component of the growth factor response. Nevertheless, the coupling between the different pathways remains to be elucidated.

The inventors report the identification of human Translokin, an FGF-2 binding protein involved in the intracellular trafficking of the internalized growth factor. Translokin fails to bind to other members of the FGF family indicating that different intracellular trafficking pathways are used by these growth factors. Surprisingly, 24 kDa FGF-2 does not interact (or very poorly) with Translokin even though the binding sites are present. This could be due to structural changes induced by the presence of an arginine-rich amino-terminal extension in HMM FGF-2 isoforms. Like FGF-2, Translokin is highly conserved among mammals and is also present in Xenopus, which is also reflected by the perfect capacity of human Translokin to interact with Xenopus FGF-2 in the two-hybrid system (data not shown). Furthermore, Translokin is ubiquitously expressed and localizes with the microtubule network. A two-hybrid screening using Translokin as bait revealed that the protein is also associated with components of microtubule apparatus. These facts, together with the presence of a putative dynactin-related domain in Translokin, reinforce the hypothesis that Translokin is the carrier of internalized exogenously added FGF-2 and belongs to a motor complex. The inventors also describe an FGF-2 mutant, Nb1a2, which is unable to interact with Translokin. Nb1a2 shows uncoupling differentiation and proliferation activities which is due to a lack in its translocation towards the nucleus. This phenotype is consistent with the results reported by Kudla and colleagues (Kudla, *et al*., 1998) who demonstrated that artificial activation of FGFR1 without FGF-2 leads to differentiation effects but not to proliferation. An hypothesis is that FGF-2 induced proliferation requires, in addition to receptor activation and sustained MAP kinases phosphorylation, an intracellular translocation of the factor to the nucleus or to the perinuclear/centrosomal compartment. Even if it is very difficult to discriminate between "nuclear" and "nucleus-associated" FGF-2, the importance of nuclear FGF-2 as a functional entity is greatly reinforced by the fact that the addition of the Large T antigen NLS to the N-terminus of Nb1a2 leads to a recovery of the mitogenic activity. Moreover, siRNA mediated inhibition of Translokin expression results in a concomitant and similar decrease of the FGF-2 nuclear translocation but has no effect on intracellular FGF-1 trafficking which is consistent with the lack of FGF-1/Translokin interaction and the inability of nocodazole to block the FGF-1 translocation (Malecki, *et al*., 2002). The inventors also observed, to a lesser extent, a reduced cytoplasmic level of FGF-2. This could suggest that Translokin participates in the uptake of FGF-2, which is the first step of its translocation. Alternatively, Translokin could also protect cytoplasmic FGF-2 from lysosomal degradation. Taken together these results clearly demonstrate that translocation of FGF-2 to the nucleus and/or its vicinity is an essential step in the mitogenic activity and that Translokin is a major component of this pathway. Bypass of this translocation pathway by the addition of a heterologous NLS to Nb1a2 suggests that the Translokin pathway is highly specific and efficient since this NLS-Nb1a2 is not able to allow more than 60% recovery of the WT FGF-2 mediated mitogenic activity. This specificity does not however exclude the possibility for Translokin to be also involved in the trafficking of other exogenous molecules. The identification of the molecular basis of the FGF-2 translocation machinery will be of great interest and could be a model for the numerous growth factors and peptide hormones that are intracellularly relocated to fully achieve their biological properties.

### EXAMPLES

### Materials and Methods

**Common molecular biology techniques**. Unless detailed, the different techniques used for the generation and production of the different constructions included in the invention are basic molecular cloning techniques, known to those skilled in the art. This includes amplification of the ADN by RT-PCR (reverse transcription coupled with PCR (Polymerase Chain Reaction)), PCR, digestion the DNA by restriction enzymes, separation of DNA fragments on agarose gels (1-2 %), ligations with DNA ligase and transformation of competent bacteria, purification of plasmid DNA from transformed bacteria. All constructs were verified by automatic sequencing.

RNAble (Eurobio, Les Ulis, France) was used to extract total RNA from culture cells or mouse organs. SuperScript II (Invitrogen, Groningen, The Netherlands) was used in the reverse transcription (RT) with following the manufacturer's instructions. Automatic nucleotide sequencing was performed using the AmpliTaq FS polymerase and an ABI 373A sequencer (Perkin Elmer Corp, Foster City, CA)

### Cloning of FGF-2 for studies in the two-hybrid system.

The following example concerns nucleotide sequences coding for bovine FGF-2, but could concern the FGF-2 from the other mammals or amphibians, since the inventors can provide data demonstrating that human Translokin (SEQ ID NO: 2) interacts equally well with the human or bovine FGF-2 as with the FGF-2 of the amphibian *Xenopus leavis* (SEQ ID NO: 24). The nucleotide sequence encoding bovine FGF-2 used in the invention was modified with regard to the wild type sequence to better correspond to the codon usage of bacterium *Escherichia coli,* in order to optimize the production of protein in prokaryotic expression systems. This cDNA was cloned in the laboratory in the vector pRF101. For the cloning in the vector aimed for the two-hybrid system (pAS2; Clontech) pRF101 was digested with *Nco*I and *Sal*I to release the fragment coding for FGF-2 18 kDa. This fragment was then cloned in the same sites of pAS2, in frame with the DNA binding domain of the yeast transcription factor GAL4.

**Cloning of FGF-1 for studies in the two-hybrid system.** Human FGF-1 was amplified by RT-PCR of 5 micrograms of total RNA extracted from SK-Hep-1 cells. For the amplification the primers 5'-GGAATTCCATATGGCTGAAGGGGAAAT-3' (sense) and 5'-ACGACGTCGACAGATCTCTTTAATCAGAAGAGACT-3' (anti-sense). The product was digested by *Nde*I and *Sal*I and cloned in the same sites of the vector pAS2.

**Generation of chimeras.** For the construction of the first series of chimeras, four junctions were chosen in beta strands 2 , 5 , 8 and 10 where the sequence is well conserved between both FGF. The nomenclature of the chimeras refer to the origin of N-terminal region ("a" for "acidic FGF" (=FGF-1) and "b " for "basic FGF" (=FGF-2)). The numbers indicate the junctions between FGF-1 and FGF-2.

A first series of eight chimeras, named Nb1-Nb4 and Na1-Na4, was thus conceived. Nb1 was generated by replacement of a 362-base pair *BamH*I/*Sal*I fragment of pAS2/FGF-2 by the corresponding sequence of the vector pAS2/FGF-1. This sequence corresponds in FGF to the sequence downstream the connection 1. In the same manner, the chimera Na1 was generated by the mutual replacement of the *BamH*I/*Sal*I fragment in pAS2/FGF-1 by the one in pAS2/FGF-2. The other chimeras were constructed by a two-step PCR approach. In the first PCR, six anti-sense primers were conceived that were overlapping junctions 2,3 and 4. Taking one of two primers in the junction 2 , "2-5a3b": (5 '-GCCAGTCTCGGTACTCTTgatagatacaacaccacg-3 ') as an example. This primer was used to generate Nb2. The sequence in the 5' portion of "2-5a3b" corresponds to the FGF-1 sequence downstream from junction 2 (capital letters in the sequence above) and the sequence in the 3' portion corresponds to the sequence upstream to the junction 2 of FGF-2 (small letters in the sequence). The primer 2-5a3b was used in PCR together with the primer pAS-sense: 5'-AAGATGCCGTCACAGATAGATTG-3' using pAS2/FGF-2 as template. The purified product was then used as a "mega-primer" with a fragment *BamH*I/*Bgl*I of pAS2 /FGF-1 as a second "mega-primer" in a PCR where the hybridization product of of two "mega-primers" constituted the template. To amplify this product, two primers flanking the FGF sequence (HA: 5 '-CCATACGATGTTCCAGATTACG-3 ' and pAS-term: 5 '-GCGACCTCATGCTATACCTGAG-3 ') were used. The final PCR product was then purified, digested by *BamH*I/*Sal*I, re-purified to finally be cloned in the same sites of pAS2/FGF-2.

**Transformation of yeast cells.** The method used to transform yeast cells with plasmid DNA is based on the protocol developed Gietz and coll. (Agatep, *et al*., 1998). A single colony of non-transformed *Saccharomyces cerevisiae* Y190 cells was grown overnight at 30°C (250 rpm) in 20 ml sterile YPD medium [1% w/v yeast extract, 2% w/v peptone, 2% w/v dextrose (glucose)]. The following day the culture was centrifuged for 5 min. at 1000g, 20°C and resuspended in 20 ml YDP medium. The optical density (OD) of the culture was determined by the measurement of an aliquot (2xdilution) in a spectrophotometer at 600nm. A new culture volume (80 ml for 20 independent transformations) with an initial OD^{600nm} of 0.3 was prepared by the addition of an appropriate volume of the overnight culture (5-8 ml for 80 ml fresh YPD). The new culture was incubated at 30°C at 250 rpm until the OD^{600nm} had reached 1.2. The cells were then harvest by centrifugation in sterile 45 ml tubes in a Beckman JS-13 swing-out rotor for 10 min. at 1000g, 20°C. The cells were washed with 25-30 ml H₂O and recentrifuged as above. Each pellet was resuspended in 1 ml 100 mM LiAc and transferred to a 1.5 ml E-tube and recentrifuged at 13000 rpm for 5-7 sec. Finally each pellet was resuspended in 100 mM LiAc to get a final volume of 250µl and the cells were subsequently distributed (25 µl) in 1.5 ml E-tubes (one per transformation). The E-tubes were centrifuged at 13000 rpm for 5-10 sec, the supernatant removed and 178 µl of TRANSFO mix and 0.5-1µg of plasmid DNA were added to each cell pellet. [TRANSFO mix (20 transformations) : 2.4 ml PEG 3350 50% (w/v), 680 µl H₂O, 360 µl 1.0 M LiAc, 100 µl denatured fish-sperm DNA (10mg/ml)]. The tubes were vortexed 1 min in a multivortex and incubated at 30°C for 30 min. The cells were then exposed to a heat shock at 42°C for 30 min. After a final centrifugation at 6500 rpm for 15 sec the supernatant was removed, the pellet resuspended in 250 µl H₂O and the yeast cells plated on selection medium (Yeast Nitrogen Base ; QBIOGEN) lacking appropriate amino acids to allow the selection of transformed cells. The colonies were allowed to grow at 30°C for 4-5 days.

**Yeast two-hybrid screening.** The two-hybrid screening was carried out as described by Van den Berghe *et al*. (Van den Berghe, *et al*., 2000). Briefly, the 18 kDa FGF-2 was used as a bait, and the corresponding cDNA was inserted in the pAS2 vector. A human placenta library (Clontech, Palo Alto, CA) cloned into the pACT2 vector was transformed in the *Saccharomyces cerevisiae* strain Y190 and screened to identify proteins that were able to interact with FGF-2.

**Measurement of β-galactosidase activity in yeast cell extract.** In order to evaluate the capacity of the chimeras to interact with Translokin, the β-galactosidase activity in yeast extracts was measured from at least 10 pooled colonies. For this the Galacto-Light kit (TROPIX Inc. Bedford, MA) was used. Breifly, the yeast cells, grown over-night in 4 ml selection medium (YNB-Trp/-Leu), were centrifuged (3 min at 13000 rpm, 20°C). The pellet was resuspended in 200 µl lysis buffer and approximately 30 µl glass beads (425-600µm; Sigma ref. G8772) were added. The cells were lysed mechanically by 2x5 min vigorous vortexing at 4°C, intercepted by a 5 min pause. To recover the cell lysat, the tubes were centrifuged for 5 min at 13000 rpm, 4°C and the supernatants transferred to fresh tubes. Twenty µl of cell extract were transferred to the well of a microtiter plate to which 80 µl of Galacton® substrate was added, previously diluted 100x in substrate dilution buffer provided by the manufacturer. In order to standardize the reaction conditions, both the substrate solution and, 60 min later, the light emission accelerator solution were added by automatic injection in a microplate luminometer (EG&G Berthold, Bad Wildbad, Germany). Immediately after injection of the accelerator solution the chemiluminescence was measured during 20 sec per sample. The obtained values were normalized for protein contents, as determined by BCA assay according to the manufacturer's instructions (Pierce Chemical CO., Rockford, IL)

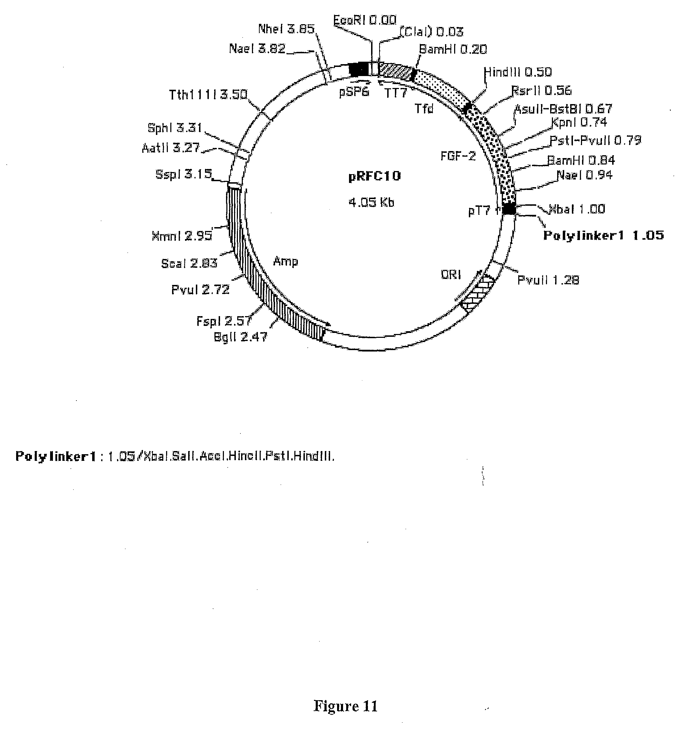

**Construction of the plasmid pRF-Nb1a2 for Nb1a2 protein expression.** The prokaryotic expression vector pRF-Nb1a2 was generated by a tri-molecular cloning approach. The plasmid pRFC10 (Fig. 11) was digested by the restriction enzymes *BamH*I and *EcoR*I to obtain a 3.21 kb DNA fragment. In parallel, the digestion of the same plasmid with the restriction enzymes *EcoR*I and *Kpn*I generated a 0.74 kb DNA fragment. pAS Nb1a2 was cleaved by the restriction enzymes *BamH*I and *Kpn*I to obtain a 0.1 kb DNA fragment. These three fragments were ligated by using the T4 DNA ligase.

**Escherichia coli transformation.** *Escherichia coli* BL21 pLys Gold (Invitrogen) were transformed by the plasmid pRF-Nb1a2. For this, 1µg of pRF-Nb1a2 was incubated on ice with 100 µl of bacteria during 10 minutes. The mix was then subjected to a heat-shock for 2 minutes at 37°C and followed by an incubation on ice for 2 minutes. 500 µl of LB was added to the mix followed by an incubation at 37°C for 45 minutes with shaking at 225-250 rpm. The reaction was then spread on a LB-ampicillin agar plate and the colonies were allowed to grow overnight at 37°C.

**Production of Nb1a2 protein.** The transformed bacteria were cultured overnight in 250 ml of LB meduim containing 2% Glucose and 100 µg/ml of Ampicillin. The overnight culture was mixed with 1 volume of prewarmed (250 ml) said medium. After 20 minutes of shaking at 37°C, the culture was mixed with 500 ml of ice cold said medium containing 4% ethanol. The culture was allowed to grow at 20°C until it obtained an OD^{600nm} of 0.6. Then IPTG was then added to the medium to a final concentration of 0.5 mM and the incubation was continued for 4 hours. The cells were then harvested by centrifugation 5000 rpm at 4°C for 10 min.

**Purification of recombinant Nb1a2.** The bacterial pellet was resuspended in 30 ml of 20 mM Tris-HCl pH 7.4. and the cells were lysed by three cycles of snap-freezing in liquid nitrogen and sonication 5x 20 seconds. The cell lysate was centrifuged 13000 rpm for 30 min at 4°C. The supernatant fraction containing the recombinant Nb1a2 was adjusted to 0.7 M NaCl and applied directly at 0.5 ml/min to an heparin-sepharose FPLC column (Amersham Pharmacia Biotech, 1 ml of resin), previously equilibrated with 20 ml of 20 mM Tris-HCl pH 7.4, 0.7 M NaCl. The column was then washed with 20 ml of equilibration buffer at 1 ml/min and then with 20 ml of buffer containing 1M NaCl. The recombinant Nb1a2 was eluted with 20 mM Tris-HCl pH 7.4, 2M NaCl and fractions of 1 ml collected. The same protocol was applied to produce other FGF proteins, tagged or not, such as bovine FGF-2, HA-FGF-1, NLS-FGF-2 and NLS-Nbla2. HA-tagged FGF-2 was kindly provided by G. Bouche (IPBS, Toulouse, France).

**Other protein production techniques.** His-tagged Translokin was produced using the IPTG-inducible pET expression system (pET-15b, Novagen, Madison, WI) in *E.coli* BL21 (DE3) pLysS and purified on a nickel-agarose column (Ni-NTA, QIAGEN, Chatsworth, CA). For equilibration and elution, 20 mM and 200 mM imidazole were used, respectively. Protein concentrations were determined by BCA assay (Pierce Chemical CO., Rockford, IL). ³⁵S-labelled FGF-2 was obtained using an *in vitro* translation system. A plasmid encoding FGF-2 was linearized, transcribed *in vitro,* and translated in a rabbit reticulocyte lysate system (RRL; Promega corp., Madison, WI) in the presence of [³⁵S]methionine (Amersham Pharmacia Biotech) as previously described (Huez, *et al*., 2001). After translation, the lysate containing [³⁵S]-FGF-2 was dialyzed against dialysis buffer (20 mM Hepes pH 7.0, 140 mM NaCl, 2 mM CaCl₂) to remove free [³⁵S]methionine and reducing agents.

**Antibodies.** Polyclonal anti-Translokin antibodies were generated through the immunization of two rabbits injected three times every 2 weeks and finally one month later with 200 µg of recombinant His-tagged Translokin, electroeluted from a polyacrylamide gel and diluted twice in Freund adjuvant (Sigma-Aldrich). Antiserum was then immunopurified with His-tagged Translokin linked to Affi gel-10 (Bio-Rad Laboratories, Hercules, CA) as previously described (Van den Berghe, *et al*., 2000). Polyclonal and monoclonal anti-HA (HA.11) were from Babco (Eurogentec, Herstal, Belgium) monoclonal anti-β-actin (AC-15), anti-β-tubulin (TUB 2.1) and peroxidase-labeled anti-His from Sigma, monoclonal anti-FGF2 (Ab3) was from Oncogene Science (Boston, MA), rabbit polyclonal against Erk2 (C14) and mouse monoclonal against the phospho p44/p42 MAPK (E10) antibodies were purchased from Cell Signaling (Beverly, MA). The horse-radish peroxidase-conjugated antibodies were from Amersham, and the FITC or Cy3-conjugated antibodies were from Sigma-Aldrich. The monoclonal anti-GAL4-DBD antibodies were from Clontech. Chromomycin A3 was from Sigma-Aldrich.

***In vitro* interaction.** A 96-well plate was coated overnight at 4°C with 10 µg/ml FGF-2, Nb1a2 or BSA. The plate was washed with PBS-tween 0.2% and 100 µl/well of blocking solution was added for 1h at 37°C. The blocking solution was discarded and the plate washed. Different concentrations of His-tagged Translokin were added for 2 h at 37°C. After three washings, horse-radish-peroxidase-conjugated anti-His antibody (1/1000) was added for 1h at 37°C. Three washings were then performed and the complexes were revealed with 100µl/well of o-phenylenediamine (4 mg/ml) containing 0.03% (v/v) hydrogen peroxide in 0.1 M citrate buffer, pH 5.0. After 20 minutes, the reactions were stopped with 50 µl of 2M H₂SO₄ and the absorbance was measured at 490 nm. When the experiment was performed in the reversed manner, the plate was coated with Translokin, and HA-tagged proteins were added. An incubation with a monoclonal anti-HA antibody (1/1000) followed by an incubation with a horse-radish-peroxidase-conjugated anti-mouse IgG antibody (1/1000) were used to reveal the interactions.

**Immunoprecipitation**. NIH-3T3 cells were transiently transfected with a plasmid encoding the full-length HA-tagged Translokin. After 48h, the cells were incubated for 2h with 60ng/ml His-tagged FGF-2. The cells were subsequently rinsed in cold PBS, scraped, pelleted, and lysed on ice in 50 mM Tris pH 7.4, 150 mM NaCl, 1% NP-40 and protease inhibitors for 30 min. Cells debris were removed by centrifugation at 10,000 x g for 10 min at 4°C. The extracts were incubated with an monoclonal anti-HA antibody coupled to protein G-Sepharose beads (Sigma) for 2h at 4°C. The beads were washed three times in the lysis buffer and the immobilized proteins were released by boiling in Laemmli buffer and then analyzed by SDS-PAGE.

**Northern blot analysis.** A human multiple-tissue Northern blot (Clontech) was probed with a 416 nucleotide fragment excised from the human Translokin cDNA by a *Xba*I digestion and subsequently labelled with [³²P]-ATP using a nick-translation kit (Promega Corp.).

**Cell culture.** Cells were grown in Dulbecco Modified Eagle Medium (DMEM, Invitrogen) containing antibiotics, 1% glutamin and serum. For COS-7 cells, the medium contained 1 g/l glucose and 5% fetal calf serum (FCS). NIH-3T3 and ABAE cells were grown in DMEM (4 g/l glucose) with 10% FCS or 10% calf serum, respectively. All cell transfections with plasmid DNA were made using Fugene 6 reagent according to the manufacturer's recommendations (Roche Diagnostics, Mannheim, Germany).

**Transfection of mammalian cells with siRNA duplexes.** The 21-nucleotide siRNA duplexes were synthesized and purified by Dharmacon Research (Boulder, CO). The siRNA sequence, targeting mouse Translokin, corresponded to the coding region 463-483 relative to the first nucleotide of the start codon. The siRNA control was the scramble one from Dharmacon. The transfection was performed with 0.1 µM of siRNA and oligofectamine reagent (Invitrogen) according to the manufacturer's instructions.

***In situ* Immunocytochemistry.** COS-7 cells, grown on coverslips, were transfected and prepared for immunofluorescence microscopy as previously described (Arnaud, *et al*., 1999). ABAE cells, grown on coverslips, were serum starved for 48h and stimulated with 20 ng/ml HA-FGF or HA-Nb1a2 for 2 hours. In some experiments cells were treated before stimulation with 10 µM nocodazole or 20 µg/ml cytochalasin B (Sigma) for 1h. Immunocytochemistry was performed as previously described (Baldin, *et al*., 1990) using polyclonal anti-HA and monoclonal anti-β-tubulin as primary antibodies and anti-rabbit IgG Cy3 conjugate (1/250) and anti-mouse IgG FITC conjugate (1/50) as secondary antibodies (Sigma). Nuclei were labeled with chromomycin A3 (0.1 mg/ml). Antibody complexes were visualized under a Leica fluorescence microscope or a confocal microscope: Zeiss LSM510 oil objective 63, operture 1.4.

**Proliferation of endothelial cells.** Five thousand ABAE (adult bovine aortic endothelial) cells were seeded in 35 mm tissue culture dishes in 1 ml of DMEM (Gibco BRL) + 10% Newborn Calf serum (Gibco BRL) + 1% Glutamin (Gibco BRL)+ 1% Amphotericin (Biochrom)+ 0.5 % gentamycin (Gibco BRL) at 37°C 10% CO₂. FGF-2 or Nb1a2, diluted in DMEM + 0.5% BSA, was added to the indicated final concentration on days 1 and 3. The cells were counted on day 5. For this the cells were washed with PBS and trypsinized in 1 ml, and counted with a Coulter Counter (Coulter Electronics Ltd).

### Organization of ABAE cells on a Basement membrane Substratum in vitro.

Twenty-four-well plates were coated with 300 µl MatriGel™ (B&D, Bedford, MA) per well, which was allowed to polymerize for 1h at 37°C. ABAE cells (2x10⁵ cells/ml) suspended in 500 µl of culture medium were then added to each well. FGF-2 or Nb1a2 were added to the wells at 0.5 ng/ml, and the plates were incubated overnight at 37°C. After removal of the medium, the culture was fixed and the length of the tube network was quantified with the Q Win Leica system.

**MAPK activation.** ABAE cells growing on 60 mm dishes (at 19,000 cells/cm²) were kept for 1 day in serum-free medium. Subsequently, cells were left untreated or stimulated with 20 ng/ml FGF-2 or Nb1a2 for the times indicated. The cells were washed twice in PBS and lysed for 15 min on ice in a HEPES-buffer (50 mM, pH 7.4) containing 150 mM NaCl, 100 mM NaF, 10 mM EDTA, 10 mM Na₄P₂O₇, 2 mM sodium orthovanadate, 1 mM PMSF, 2 mg/ml aprotinin, 20 mM leupeptin and 1% Triton. The mixture was gently agitated for 15 min at 4°C and centrifuged at 13,000g for 15 min. Soluble proteins (60 µg) were separated on a 12.5 % SDS-polyacrylamide gel and subjected to immunoblotting with the indicated antibodies.

**ABAE fractionation.** The fractionation was performed as previously described (Baldin, *et al*., 1990). Purity and integrity of preparations of nuclei (prior to sonication) were monitored by phase contrast microscopy, and by the quantitative analysis of the cytoplasmic Lactate Dehydrogenase (LDH) content.

**Animal studies.** All procedures involving experimental animals were performed in accordance with the recommendations of the French Accreditation of Laboratory Animal Care. The mice were housed in stainless-steel cages in groups of 5, kept in a temperature-controlled facility on a 12-h light-dark cycle, and fed normal laboratory mouse chow diet. For all surgical procedures, mice were anesthetized by intraperitoneal injection of 150 mg/kg ketamine and allowed to recover on a thermostated pack. C57BL/6 mice were divided into three groups (n=10 each) for treatment with either FGF-2, Nb1a2 or placebo (saline). FGF-2 and Nb1a2 were administrated by bolus of 5µg IV in the jugular vein, just after electric carotid injury.

**Electric injury model.** The inventors adapted the electric injury model described by Carmeliet *et al*. (Carmeliet, *et al*., 1997) on the femoral artery to the common carotid artery, the latter being easier to dissect. Electric carotid artery injury was performed in 6-week-old mice. Surgery was carried out using a dissection microscope (Nikon SMZ-2B) in six-week-old female mice weighing 20 g on average. Since the proximal part of the carotid artery is intrathoracic, the injury could not be applied to the whole common carotid artery. The left common carotid artery was exposed via an anterior incision of the neck. The electric injury (mainly thermic) was applied to the distal part of the common carotid artery. The carotid artery was injured by electric current using a bipolar microregulator. In order to standardize the temperature increase in the vessel wall, we used forceps with large tips (1mm), instead of microsurgical forceps (200 µm) and a bipolar microregulator Force FX (Valleylab®). The "precise" mode of this apparatus allowed delivery of electric energy within a narrow range of resistance, as the generator microprocessor disrupted the electric current when the resistance increased as a consequence of temperature increase. This enabled the increase in tissue temperature to be controlled, and avoided the risk of desiccation and coagulation of the arterial wall. The optimal conditions were determined as follows: electric current of 2 Watts applied for 2 seconds to each mm of carotid artery over a total length of 4mm with the help of a size marker placed parallel to the long axis of the carotid. Despite optimization of the technique, coagulation and thrombosis of the carotid artery occurred in approximately 10 % of the cases, which were then excluded from the study.

**Evaluation of Reendothelialization.** Three days following the injury, the endothelial regeneration process was evaluated by staining the denuded areas with Evans blue dye. For this, 50µl of solution containing 5% Evans blue diluted in saline was injected in the tail vein using a 30-gauge needle 10 minutes before sacrifice, followed by fixation using a perfusion with 4% phosphate-buffered formalin (pH 7.0) for 5 minutes. Blood, saline and fixative was removed through an incision in the right atrium. The left common carotid artery was dissected with an adjacent portion of the aortic arch and carotid bifurcation. The artery was then opened longitudinaly and placed between slides with Fluoprep®. After transparency scanning and numerization, the total and stained carotid artery area were planimetered using an "image analyzer" (VISIOL@b™2000). The ratio between the area stained in blue and the total carotid artery area was calculated. The surface of area remaining deendothelialized was indexed to the total carotid artery area to take into account the changes in vessel area due to both the elasticity of the carotid artery and to the flattening of the vessel between slides. Results are expressed as means ± S.E.M. ANOVA was used for the comparison of the treatments with FGF-2 and Nb1a2 compared to controls.

**Morphological studies** Ten days following injury, carotids were removed and fixed in 3.7% paraformaldehyde. The samples were dehydrated with ethanol and Microclearing™ (Microstain, Granrolo, Italy), and then embedded in paraffin, Sections (6 µm) were rehydrated and stained with hematoxylin to visualize the nuclei.

Silver nitrate staining was used to examine the morphology of the regenerated endothelium, 10 days after carotid electric injury (n=3). Left ventricle was perfused immediately after death with solutions of 5% dextrose, 0.25% AgNO₃, 1% NH₄Br, and 3% CoBr each for 1 minute, followed by perfusion of 100 ml buffered formalin. The carotid artery was excised, immersed in formalin for 1 hour, and rinsed in 0.1 M sodium cacodylate buffer. Control and injured carotids were incised longitudinally, dehydrated and examined by light microscopy.

### References

Agatep, R., Kirkpatrick, R. D., Parchaliuk, D. L., Woods, R. A., and Gietz, R. D. (1998) Transformation of Saccharomyces cerevisiae by the lithium acetate/single-stranded carrier DNA/polyethylene glycol (LiAc/ss-DNA/PEG) protocol. *Technical Tips Online* http://tto.trends.com

Arese, M., Chen, Y., Florkiewicz, R. Z., Gualandris, A., Shen, B., and Rifkin, D. B. (1999) Nuclear activities of basic fibroblast growth factor: potentiation of low-serum growth mediated by natural or chimeric nuclear localization signals. *Mol Biol Cell* 10, 1429-1444.

Arnaud, E., Touriol, C., Boutonnet, C., Gensac, M. C., Vagner, S., Prats, H., and Prats, A. C. (1999) A new 34-kilodalton isoform of human fibroblast growth factor 2 is cap dependently synthesized by using a non-AUG start codon and behaves as a survival factor. *Mol Cell Biol* 19, 505-514.

Bailly, K., Soulet, F. , Leroy, D., Amalric, F., and Bouche, G. (2000) Uncoupling of cell proliferation and differentiation activities of basic fibroblast growth factor. *FASEB J* 14, 333-344.

Baldin, V., Roman, A. M., Bosc-Bierne, I., Amalric, F., and Bouche, G. (1990) Translocation of bFGF to the nucleus is G1 phase cell cycle specific in bovine aortic endothelial cells. *EMBO J* 9, 1511-1517.

Bennett, M. R., and O'Sullivan, M. (2001) Mechanisms of angioplasty and stent restenosis: implications for design of rational therapy. *Pharmacol Ther* 91, 149-166.

Bikfalvi, A., Klein, S., Pintucci, G., Quarto, N., Mignatti, P., and Rifkin, D. B. (1995) Differential modulation of cell phenotype by different molecular weight forms of basic fibroblast growth factor: possible intracellular signaling by the high molecular weight forms. *J Cell Biol* 129, 233-243.

Bikfalvi, A., Klein, S., Pintucci, G., and Rifkin, D. B. (1997) Biological roles of fibroblast growth factor-2. *Endocr Rev* 18, 26-45.

Bonnet, H., Filhol, O., Truchet, I., Brethenou, P., Cochet, C., Amalric, F., and Bouche, G. (1996) Fibroblast growth factor-2 binds to the regulatory beta subunit of CK2 and directly stimulates CK2 activity toward nucleolin. *J Biol Chem 271,* 24781-24787.

Bouche, G., Gas, N., Prats, H., Baldin, V., Tauber, J. P., Teissie, J., and Amalric, F. (1987) Basic fibroblast growth factor enters the nucleolus and stimulates the transcription of ribosomal genes in ABAE cells undergoing G0----G1 transition. *Proc Natl Acad Sci U S A* 84, 6770-6774.

Brouchet, L., Krust, A., Dupont, S., Chambon, P., Bayard, F., and Arnal, J. F. (2001) Estradiol accelerates reendothelialization in mouse carotid artery through estrogen receptor-alpha but not estrogen receptor-beta. *Circulation* 103, 423-428.

Bugler, B., Amalric, F., and Prats, H. (1991) Alternative initiation of translation determines cytoplasmic or nuclear localization of basic fibroblast growth factor. *Mol Cell Biol* 11, 573-577.

Carmeliet, P., Moons, L., Stassen, J. M., De Mol, M., Bouche, A., van den Oord, J. J., Kockx, M., and Collen, D. (1997) Vascular wound healing and neointima formation induced by perivascular electric injury in mice. *Am J Pathol* 150, 761-776.

Celletti, F. L., Waugh, J. M., Amabile, P. G., Brendolan, A., Hilfiker, P. R., and Dake, M. D. (2001) Vascular endothelial growth factor enhances atherosclerotic plaque progression. *Nat Med* 7, 425-429.

Clarke, W. E., Berry, M., Smith, C., Kent, A., and Logan, A. (2001) Coordination of fibroblast growth factor receptor 1 (FGFR1) and fibroblast growth factor-2 (FGF-2) trafficking to nuclei of reactive astrocytes around cerebral lesions in adult rats. *Mol Cell Neurosci* 17, 17-30.

Couderc, B., Prats, H., Bayard, F., and Amalric, F. (1991) Potential oncogenic effects of basic fibroblast growth factor requires cooperation between CUG and AUG-initiated forms. *Cell Regulation* 2, 709-718

Cross, M. J., and Claesson-Welsh, L. (2001) FGF and VEGF function in angiogenesis: signalling pathways, biological responses and therapeutic inhibition. *Trends Pharmacol Sci* 22, 201-207.

Darblade, B., Pendaries, C., Krust, A., Dupont, S., Fouque, M. J., Rami, J., Chambon, P., Bayard, F., and Arnal, J. F. (2002) Estradiol alters nitric oxide production in the mouse aorta through the alpha-, but not beta-, estrogen receptor. *Circ Res* 90, 413-419.

Davies, M. J., Mitchell, C. A., Maley, M. A., Grounds, M. D., Harvey, A. R., Plant, G. W., Wood, D. J., Hong, Y., and Chirila, T. V. (1997) In vitro assessment of the biological activity of basic fibroblast growth factor released from various polymers and biomatrices. *J Biomater Appl* 12, 31-56.

Degertekin, M., Serruys, P. W., Foley, D. P., Tanabe, K., Regar, E., Vos, J., Smits, P. C., van der Giessen, W. J., van den Brand, M., de Feyter, P., and Popma, J. J. (2002) Persistent inhibition of neointimal hyperplasia after sirolimus-eluting stent implantation: long-term (up to 2 years) clinical, angiographic, and intravascular ultrasound follow-up. *Circulation* 106, 1610-1613.

Desgranges, P., Caruelle, J. P., Carpentier, G., Barritault, D., and Tardieu, M. (2001) Beneficial use of fibroblast growth factor 2 and RGTA, a new family of heparan mimics, for endothelialization of PET prostheses. *J Biomed Mater Res* 58, 1-9

Dixit, P., Hern-Anderson, D., Ranieri, J., and Schmidt, C. E. (2001) Vascular graft endothelialization: comparative analysis of canine and human endothelial cell migration on natural biomaterials. *J Biomed Mater Res* 56, 545-555.

Elbashir, S. M., Harborth, J., Lendeckel, W., Yalcin, A., Weber, K., and Tuschl, T. (2001) Duplexes of 21-nucleotide RNAs mediate RNA interference in cultured mammalian cells. *Nature* 411, 494-498.

Estival, A., Monzat, V., Miquel, K., Gaubert, F., Hollande, E., Korc, M., Vaysse, N., and Clemente, F. (1996) Differential regulation of fibroblast growth factor (FGF) receptor-1 mRNA and protein by two molecular forms of basic FGF. Modulation of FGFR-1 mRNA stability. *J Biol Chem* 271, 5663-5670.

Farb, A., Heller, P. F., Shroff, S., Cheng, L., Kolodgie, F. D., Carter, A. J., Scott, D. S., Froehlich, J., and Virmani, R. (2001) Pathological analysis of local delivery of paclitaxel via a polymer-coated stent. *Circulation* 104, 473-479.

Fischman, D. L., Leon, M. B., Baim, D. S., Schatz, R. A., Savage, M. P., Penn, I., Detre, K., Veltri, L., Ricci, D., Nobuyoshi, M., and et al. (1994) A randomized comparison of coronary-stent placement and balloon angioplasty in the treatment of coronary artery disease. Stent Restenosis Study Investigators. *N Engl J Med* 331, 496-501.

Florkiewicz, R. Z., and Sommer, A. (1989) Human basic fibroblast growth factor gene encodes four polypeptides: three initiate translation from non-AUG codons. *Proc Natl Acad Sci U S A* 86, 3978-3981.

Friesel, R. E., and Maciag, T. (1995) Molecular mechanisms of angiogenesis: fibroblast growth factor signal transduction. *FASEB J* 9, 919-925.

George, J. N. (2000) Platelets. *Lancet* 355, 1531-1539.

Gleizes, P. E., Noaillac-Depeyre, J., Amalric, F., and Gas, N. (1995) Basic fibroblast growth factor (FGF-2) internalization through the heparan sulfate proteoglycans-mediated pathway: an ultrastructural approach. *Eur J Cell Biol* 66, 47-59.

Gleizes, P. E., Noaillac-Depeyre, J., Dupont, M. A., and Gas, N. (1996) Basic fibroblast growth factor (FGF-2) is addressed to caveolae after binding to the plasma membrane of BHK cells. *Eur J Cell Biol* 71, 144-153.

Grieb, T. A., and Burgess, W. H. (2000) The mitogenic activity of fibroblast growth factor-1 correlates with its internalization and limited proteolytic processing. *J Cell Physiol* 184, 171-182.

Hawker, J. R., Jr., and Granger, H. J. (1992) Internalized basic fibroblast growth factor translocates to nuclei of venular endothelial cells. *Am J Physiol* 262, H1525-1537.

Heldman, A. W., Cheng, L., Jenkins, G. M., Heller, P. F., Kim, D. W., Ware, M., Jr., Nater, C., Hruban, R. H., Rezai, B., Abella, B. S., Bunge, K. E., Kinsella, J. L., Sollott, S. J., Lakatta, E. G., Brinker, J. A., Hunter, W. L., and Froehlich, J. P. (2001) Paclitaxel stent coating inhibits neointimal hyperplasia at 4 weeks in a porcine model of coronary restenosis. *Circulation* 103, 2289-2295.

Huez, I., Bornes, S., Bresson, D., Creancier, L., and Prats, H. (2001) New vascular endothelial growth factor isoform generated by internal ribosome entry site-driven CUG translation initiation. *Mol Endocrinol* 15, 2197-2210.

Imamura, T., Engleka, K., Zhan, X., Tokita, Y., Forough, R., Roeder, D., Jackson, A., Maier, J. A., Hla, T., and Maciag, T. (1990) Recovery of mitogenic activity of a growth factor mutant with a nuclear translocation sequence. *Science* 249, 1567-1570.

Ishihara, M., Sato, M., Hattori, H., Saito, Y., Yura, H., Ono, K., Masuoka, K., Kikuchi, M., Fujikawa, K., and Kurita, A. (2001) Heparin-carrying polystyrene (HCPS)-bound collagen substratum to immobilize heparin-binding growth factors and to enhance cellular growth. *J Biomed Mater Res* 56, 536-544.

Jackson, C. L., and Reidy, M. A. (1993) Basic fibroblast growth factor: its role in the control of smooth muscle cell migration. *Am J Pathol* 143, 1024-1031

Jans, D. A. (1994) Nuclear signaling pathways for polypeptide ligands and their membrane receptors? *FASEB J* 8, 841-847.

Johnson, D. E., and Williams, L. T. (1993) Structural and functional diversity in the FGF receptor multigene family. *Adv Cancer Res* 60, 1-41.

Joy, A., Moffett, J., Neary, K., Mordechai, E., Stachowiak, E. K., Coons, S., Rankin-Shapiro, J., Florkiewicz, R. Z., and Stachowiak, M. K. (1997) Nuclear accumulation of FGF-2 is associated with proliferation of human astrocytes and glioma cells. *Oncogene* 14, 171-183.

Kanda, S., Hodgkin, M. N., Woodfield, R. J., Wakelam, M. J., Thomas, G., and Claesson-Welsh, L. (1997) Phosphatidylinositol 3'-kinase-independent p70 S6 kinase activation by fibroblast growth factor receptor-1 is important for proliferation but not differentiation of endothelial cells. *J Biol Chem* 272, 23347-23353.

Keresztes, M., and Boonstra, J. (1999) Import(ance) of growth factors in(to) the nucleus. *J Cell Biol* 145, 421-424.

Kolpakova, E., Wiedlocha, A., Stenmark, H., Klingenberg, O., Falnes, P. O., and Olsnes, S. (1998) Cloning of an intracellular protein that binds selectively to mitogenic acidic fibroblast growth factor. *Biochem J* 336, 213-222.

Kudla, A. J., Jones, N. C., Rosenthal, R. S., Arthur, K., Clase, K. L., and Olwin, B. B. (1998) The FGF receptor-1 tyrosine kinase domain regulates myogenesis but is not sufficient to stimulate proliferation. *J Cell Biol* 142, 241-250.

Letourneur, D., Machy, D., Pelle, A., Marcon-Bachari, E., D'Angelo, G., Vogel, M., Chaubet, F., and Michel, J. B. (2002) Heparin and non-heparin-like dextrans differentially modulate endothelial cell proliferation: in vitro evaluation with soluble and crosslinked polysaccharide matrices. *J Biomed Mater Res* 60, 94-100

Lindner, V., Majack, R. A., and Reidy, M. A. (1990) Basic fibroblast growth factor stimulates endothelial regrowth and proliferation in denuded arteries. *J Clin Invest* 85, 2004-2008

Lindner, V., Lappi, D. A., Baird, A., Majack, R. A., and Reidy, M. A. (1991) Role of basic fibroblast growth factor in vascular lesion formation. *Circ Res* 68, 106-113

Lindner, V., and Reidy, M. A. (1991) Proliferation of smooth muscle cells after vascular injury is inhibited by an antibody against basic fibroblast growth factor. *Proc Natl Acad Sci U S A* 88, 3739-3743

Malecki, J., Wiedlocha, A., Wesche, J., and Olsnes, S. (2002) Vesicle transmembrane potential is required for translocation to the cytosol of externally added FGF-1. *EMBO J* 21, 4480-4490.

Mason, I. J. (1994) The ins and outs of fibroblast growth factors. *Cell* 78, 547-552.

Morice, M. C., Serruys, P. W., Sousa, J. E., Fajadet, J., Ban Hayashi, E., Perin, M., Colombo, A., Schuler, G., Barragan, P., Guagliumi, G., Molnar, F., and Falotico, R. (2002) A randomized comparison of a sirolimus-eluting stent with a standard stent for coronary revascularization. *N Engl J Med* 346, 1773-1780.

Nagase, T., Miyajima, N., Tanaka, A., Sazuka, T., Seki, N., Sato, S., Tabata, S., Ishikawa, K., Kawarabayasi, Y., Kotani, H., and et al. (1995) Prediction of the coding sequences of unidentified human genes. III. The coding sequences of 40 new genes (KIAA0081-KIAA0120) deduced by analysis of cDNA clones from human cell line KG-1. *DNA Res* 2, 37-43.

Nakatake, Y., Hoshikawa, M., Asaki, T., Kassai, Y., and Itoh, N. (2001) Identification of a novel fibroblast growth factor, FGF-22, preferentially expressed in the inner root sheath of the hair follicle. *Biochim Biophys Acta* 1517, 460-463.

Okada-Ban, M., Moens, G., Thiery, J. P., and Jouanneau, J. (1999) Nuclear 24 kD fibroblast growth factor (FGF)-2 confers metastatic properties on rat bladder carcinoma cells. *Oncogene* 18, 6719-6724.

Ornitz, D. M., and Itoh, N. (2001) Fibroblast growth factors. *Genome Biol 2,* REVIEWS3005.

Pages, G., Lenormand, P., L'Allemain, G., Chambard, J. C., Meloche, S., and Pouyssegur, J. (1993) Mitogen-activated protein kinases p42mapk and p44mapk are required for fibroblast proliferation. *Proc Natl Acad Sci U S A* 90, 8319-8323.

Patry, V., Arnaud, E., Amalric, F., and Prats, H. (1994) Involvement of basic fibroblast growth factor NH2 terminus in nuclear accumulation. *Growth Factors* 11, 163-174

Patry, V., Bugler, B., Maret, A., Potier, M., and Prats, H. (1997) Endogenous basic fibroblast growth factor isoforms involved in different intracellular protein complexes. *Biochem J* 326, 259-264.

Prats, H., Kaghad, M., Prats, A. C., Klagsbrun, M., Lelias, J. M., Liauzun, P., Chalon, P., Tauber, J. P., Amalric, F., Smith, J. A., and et al. (1989) High molecular mass forms of basic fibroblast growth factor are initiated by alternative CUG codons. *Proc Natl Acad Sci U S A* 86, 1836-1840.

Quarto, N., Finger, F. P., and Rifkin, D. B. (1991) The NH2-terminal extension of high molecular weight bFGF is a nuclear targeting signal. *J Cell Physiol* 147, 311-318.

Regar, E., Sianos, G., and Serruys, P. W. (2001) Stent development and local drug delivery. *Br Med Bull* 59, 227-248

Reiland, J., and Rapraeger, A. C. (1993) Heparan sulfate proteoglycan and FGF receptor target basic FGF to different intracellular destinations. *J Cell Sci* 105, 1085-1093.

Reilly, J. F., and Maher, P. A. (2001) Importin beta-mediated nuclear import of fibroblast growth factor receptor: role in cell proliferation. *J Cell Biol* 152, 1307-1312.

Roghani, M., and Moscatelli, D. (1992) Basic fibroblast growth factor is internalized through both receptor-mediated and heparan sulfate-mediated mechanisms. *J Biol Chem* 267, 22156-22162.

Serruys, P. W., de Jaegere, P., Kiemeneij, F., Macaya, C., Rutsch, W., Heyndrickx, G., Emanuelsson, H., Marco, J., Legrand, V., Materne, P., and et al. (1994) A comparison of balloon-expandable-stent implantation with balloon angioplasty in patients with coronary artery disease. Benestent Study Group. *N Engl J Med* 331, 489-495.

Shen, B., Arese, M., Gualandris, A., and Rifkin, D. B. (1998) Intracellular association of FGF-2 with the ribosomal protein L6/TAXREB107. *Biochem Biophys Res Commun* 252, 524-528.

Skjerpen, C. S., Nilsen, T., Wesche, J., and Olsnes, S. (2002) Binding of FGF-1 variants to protein kinase CK2 correlates with mitogenicity. *EMBO J* 21, 4058-4069

Stachowiak, E. K., Maher, P. A., Tucholski, J., Mordechai, E., Joy, A., Moffett, J., Coons, S., and Stachowiak, M. K. (1997) Nuclear accumulation of fibroblast growth factor receptors in human glial cells--association with cell proliferation. *Oncogene* 14, 2201-2211.

Tanihara, M., Suzuki, Y., Yamamoto, E., Noguchi, A., and Mizushima, Y. (2001) Sustained release of basic fibroblast growth factor and angiogenesis in a novel covalently crosslinked gel of heparin and alginate. *J Biomed Mater Res* 56, 216-221.

Tassiopoulos, A. K., and Greisler, H. P. (2000) Angiogenic mechanisms of endothelialization of cardiovascular implants: a review of recent investigative strategies. *J Biomater Sci Polym Ed* 11, 1275-1284

Van Belle, E., Bauters, C., Asahara, T., and Isner, J. M. (1998) Endothelial regrowth after arterial injury: from vascular repair to therapeutics. *Cardiovasc Res* 38, 54-68.

Van den Berghe, L., Laurell, H., Huez, I., Zanibellato, C., Prats, H., and Bugler, B. (2000) FIF [fibroblast growth factor-2 (FGF-2)-interacting-factor], a nuclear putatively antiapoptotic factor, interacts specifically with FGF-2. *Mol Endocrinol* 14, 1709-1724.

Walicke, P. A., and Baird, A. (1991) Internalization and processing of basic fibroblast growth factor by neurons and astrocytes. *J Neurosci* 11, 2249-2258.

Wiedlocha, A., Falnes, P. O., Madshus, I. H., Sandvig, K., and Olsnes, S. (1994) Dual mode of signal transduction by externally added acidic fibroblast growth factor. *Cell* 76, 1039-1051.

Yamaguchi, T. P., and Rossant, J. (1995) Fibroblast growth factors in mammalian development. *Curr Opin Genet Dev* 5, 485-491.

Zhu, X., Komiya, H., Chirino, A., Faham, S., Fox, G. M., Arakawa, T., Hsu, B. T., and Rees, D. C. (1991) Three-dimensional structures of acidic and basic fibroblast growth factors. *Science* 251, 90-93.

### Figure 1: Characterization of Translokin, an FGF-2 interacting protein

Alignment of the predicted amino acid sequences of human, mouse and bovine Translokin. The cDNAs were cloned from a human placenta two-hybrid library, mice embryos and ABAE (bovine) cells, respectively. The arrows indicate the 5' end of each clone (A,B,C,D) isolated by the two-hybrid system.

### Figure 2: Specific and direct interaction between Translokin and FGF-2

**a,** The specificity of the interaction between Translokin and FGF-2 was first tested in the yeast two-hybrid system. Several FGFs, including the 18 kDa and 24 kDa forms of FGF-2, and Translokin were fused to DNA binding (DB) or activating (AD) domains of Gal4 and subjected to a yeast two hybrid assay to evaluate a potential interaction.
**b,** Direct interaction between FGF-2 and Translokin by ELISA : FGF-2 and BSA were coated on a 96-well plate and incubated with different concentration of His-tagged recombinant Translokin. The association was revealed using a horse-radish-peroxidase-conjugated antibody against the (His)₆ tag, then read at 490 nm with an ELISA microplate reader. Data are the mean ±S.D. of triplicate samples.
**c,** The specificity of the interaction between Translokin and FGF-2 was tested in an ELISA assay. Translokin was coated on a 96-well plate and incubated with HA-tagged 18 kDa FGF-2, FGF-1, or 24 kDa FGF-2. The association was revealed using a monoclonal anti-HA antibody and a horse-radish peroxidase-conjugated anti-mouse IgG antibody. The results were read at 490 nm with an ELISA microplate reader. Data are the mean ±S.D. of triplicate samples.
**d,** Co-immunoprecipitation of HA-tagged Translokin and FGF-2. Mouse NIH-3T3 cells were transiently transfected or not (-) with a plasmid encoding the full-length HA-tagged Translokin and incubated during 2h with 60 ng/ml His-tagged FGF-2 (18kDa). Extracts from these cells were immunoprecipitated with monoclonal anti-HA coupled to protein G-Sepharose beads. Immunoprecipitated proteins treated under non-reducing conditions were subjected to Western blotting and revealed with monoclonal anti-HA and anti-His antibodies.
**e,** Co-immunoprecipitation of Translokin and FGF-2. Extracts from NIH-3T3 cells stimulated (+) or not (-) with 60 ng/ml HA-tagged FGF-2 (18kDa) were subjected to immunoprecipitation with anti-Translokin coupled to protein A-Sepharose beads or with a control antibody. Immunoprecipitated proteins were subjected to Western blotting and revealed with monoclonal anti-HA to detect HA-tagged FGF-2.

### Figure 3: Translokin expression is ubiquitous

**a,** Northern blot (MTN™, Clontech) analysis showing an ubiquitous expression of Translokin in different human tissues.
**b,** Expression of Translokin mRNA was detected by RT-PCR on mRNA from different cell lines. Ctrl+ is a positive control that corresponded to a PCR done with plasmid containing the Translokin cDNA as the template. Ctrl- is a negative control and corresponds to a PCR done without added template.
**c,** Immunolocalization of Translokin in transfected COS-7 cells. Transfection was performed with a plasmid encoding the full-length Translokin. Cells were stained using anti-Translokin (top) or polyclonal anti-HA (bottom) followed by the anti-rabbit-Cy3 conjugated antibody and the monoclonal anti-β-tubulin followed by the anti-mouse-FITC conjugated antibody.

### Figure 4: Interaction of FGF-2 / FGF-1 chimeras with Translokin

**a,** Two-hybrid system. Relative intensity of the interactions between FGF-1/2 chimeras and Translokin measured by β-galactosidase activity in yeast extracts. Four junctions were chosen in the well-conserved β-strands 2,5,8 and 10. The regions derived from FGF-1 are represented in white and those from FGF-2 are in black. The nomenclature is based on the origin of the N-terminal extremity; "a" for acidic FGF (FGF-1) and "b" for basic FGF (FGF-2). The numbers indicate the junctions between FGF-1 and FGF-2. The data shown are the mean ±S.E.M from at least three independent transformations. β-galactosidase assays were performed in duplicates.
**b,** Analysis of the expression of chimeras/GAL4-DBD fusion proteins. Yeast extracts were subjected to Western blotting and revealed with a monoclonal anti-GAL4-DBD antibody.
**c,** 10 µg/ml of FGF-2, Nb1a2 or BSA were immobilized on a 96-well plate and the assay was performed as in figure 2b with 10 µg/ml of His-tagged Translokin. Data are the mean ±S.D. of triplicate samples.

### Figure 5: Characterization of Nb1a2

**a,** Proliferation assay. ABAE cells were stimulated by different concentrations of FGF-2 or Nb1a2 added at day 1 and 3. At day 5 cells were trypsinized and counted with a Coulter counter^{R} ZM. Data are the mean ±S.D of triplicate samples. The experiment was repeated at least three times with similar results.
**b,** Organization of ABAE cells on a Basement Membrane Substratum *in vitro.* ABAE cells were plated on MATRIGEL™ coated wells in presence of 0.5 ng/ml FGF-2 or Nb1a2 during 12 hours. Tube formation was observed and quantified with a Leica Q Win system.
**c,** Relative receptors binding affinity : confluent ABAE cells were incubated for 2h at 4°C with [³⁵S]-FGF-2 and increasing concentrations of unlabeled FGF-2 or Nb1a2. Cells were then treated as previously described (Estival, *et al*., 1996). Values represent the percentage of specific binding of [³⁵S]-FGF-2 on FGF receptors determined at different concentrations of competitors (D: 10 µg of denatured competitors).
d, Recombinant FGF-2 and Nb1a2 were purified by heparin-sepharose chromatography. Elution was performed using a NaCl gradient. Fractions were analyzed by SDS-PAGE and immunoblotting with an anti-FGF-2 antibody.
**e,** Effect of FGFs on MAP kinase activation : G0-arrested ABAE cells were stimulated (or not) with 20 ng/ml FGF-2 or Nb1a2 during different periods. Cells were then harvest and MAPK activation was detected by Western blotting using a monoclonal antibody raised against phosphorylated forms of p44 and p42. Immunoblotting with an antibody raised against the non-phosphorylated form of p44 and p42 was performed to verify that equal amounts of cell extracts had been used.

### Figure 6: FGF-2 intracellular localization

**a,** G0-arrested ABAE cells were incubated with or without 20ng/ml HA-FGF-2 or HA-Nb1a2 (20 ng/ml or 100 ng/ml) for 2h. The cells were lysed and nuclear and postnuclear fractions were incubated in the presence of heparin sepharose beads. Proteins bound to heparin sepharose beads were analysed by SDS-PAGE and immunoblotting using monoclonal α-HA antibody.
**b, Confocal microscopy analysis.** ABAE cells were stimulated (B-F) or not (A), by HA-FGF-2 (B-E) or HA-Nb1a2 (F) during 2h at 37°C. In some cases, cells were pretreated, with nocodazole (10µM) (C) or cytochalasin B (20µg/ml) (E) 1h before HA-FGF-2 stimulation. After HA-FGF-2 incubation, cells were fixed and stained with polyclonal anti-HA (HA-FGF-2, HA-Nb1a2), monoclonal anti-β-tubulin (A-C, F) or anti-β-actin (D, E) antibodies. Nuclei were labeled with Chromomycin A3.

### Figure 7: Recovery of the mitogenic activity of Nb1a2 and inhibition of FGF-2

**a,** Proliferation assay. ABAE cells were stimulated by different concentrations of FGF-2, NLS-FGF-2, Nb1a2 or NLS-Nb1a2 added at day 1 and 3. At day 5 cells were trypsinized and counted with a Coulter counter ^{R} ZM. The experiment was repeated at least three times with similar results, using recombinant proteins from two independent productions.
**b,** HA-NLS-FGF-2 and HA-NLS-Nbla2 are translocated to the nucleus. After stimulation with (+) or without (-) 60 ng/ml NLS-tagged growth factors, NIH-3T3 cells were treated and fractionated as described for figure 6a. Nuclear and postnuclear fractions were analyzed by SDS-PAGE and immunoblotting using monoclonal anti-HA antibody.
**c,** Western-blot for Translokin expression in scramble siRNA (lane 1) and Translokin siRNA (lane 2) transfected NIH-3T3 using an anti-Translokin antibody (top panel). β-actin expression was used as a loading control (bottom panel)
**d,** Translokin siRNA inhibits FGF-2 translocation. Scramble siRNA (lane 1) and Translokin siRNA (lane 2) transfected NIH-3T3 were stimulated with 20ng/ml HA-FGF-2 for 2h. The cells were then treated and fractionated as described for figure 6a. Nuclear and postnuclear fractions were analyzed by SDS-PAGE and immunoblotting using monoclonal anti-HA antibody. Protein levels were quantified by densitometry of immunoblots, and values normalized to control siRNA transfected cells. Values represent the mean ± SD of triplicate experiments.
**e,** Translokin siRNA does not affect FGF-1 translocation. Scramble siRNA (lane 2) and Translokin siRNA (lane 3) transfected NIH-3T3 were stimulated with (lanes 2 and 3) or without (lane 1) 60ng/ml HA-FGF-1 and 10 u/ml of heparin during 5h. The cells were then treated as described in figure 7d.

### Figure 8: FGF-2 and Nb1a2 accelerate the process of reendothelialisation.

Four millimeters of the distal part of the common carotid artery of C57/B16 mice was injured with a bipolar electrosurgical generator (Valleylab Force FX) as described in (Brouchet, *et al*., 2001). The bars (mean ±SEM) indicate the reendothelialized surface at day +3 as percentage of the initially injuried surface after one singel intraveneous administration of 5µg of FGF-2 or Nb1a2. (controls (n=7); FGF-2 and Nb1a2 (n=8)).

### Figure 9: Morphology of reendothelialized and non-injured carotids.

Silver nitrate staining of carotids 10 days after electric injury from (a) FGF-2 treated (5µg administrated intravenously (IV) in bolus), (b) Nb1a2 treated (5µg IV in bolus) and (c) non-injured animals. After perfusion with silver nitrate solution and buffered formalin, the carotid artery was excised, immersed in formalin, and rinsed in cacodylate buffer. The carotids were incised longitudinally, dehydrated and examined by light microscopy.

### Figure 10: Detailed analysis of the first Translokin-interacting region in FGF-2

The capacity of nine targeted mutants in the most N-terminal Translokin-interacting region in FGF-2 to interact with Translokin. The interaction studies were performed in the yeast two-hybrid system as described in the materials and methods section. The results (mean ± SEM) are represented as the percentage of the beta-galactosidase activity obtained with FGF-2 and Translokin.

### Figure 11: Map of the plasmid pRFC10

Map of the plasmid pRFC10 used for the construction of pRF-Nb1a2 for the production of recombinant Nb1a2. The cassette BamHI/KpnI in pRFC10 was exchanged by the same fragment in pAS2-Nb1a2.

## Claims

1. Use of animal or human basic fibroblast growth factor (FGF-2) derived proteins for the preparation of biomaterials or medical devices chosen among medical prostheses, such as endovascular stents, coated endoprostheses or by-pass grafts, said FGF-2 derived proteins being chosen among FGF-2 mutants which are unable to interact specifically with the Translokin represented by SEQ ID NO: 2, i.e. having a binding capacity to said Translokin of less than 40%, and more preferably of less than 10%, said FGF-2 mutants being such that they are defective in intracellular trafficking and in stimulating the proliferation of endothelial cells, and that their tubulogenesis activity is preserved.

2. Use according to claim 1, of FGF-2 mutants corresponding to bovine FGF-2 represented by SEQ ID NO: 4, or to human FGF-2 represented by SEQ ID NO: 6, and wherein the region delimited by the amino acids located from positions 40 to 130 contains at least one mutation by deletion, or substitution by a heterologous amino acid natural or not, of at least one amino acid of said region, or by insertion of at least one heterologous amino acid natural or not in said region.

3. Use according to claim 1 or 2, of FGF-2 mutants corresponding to FGF-2 represented by SEQ ID NO: 4, or by SEQ ID NO: 6, wherein the region delimited by the amino acids located from positions 40 to 75, and/or the region delimited by the amino acids located from positions 105 to 130, contain at least one mutation as defined in claim 2.

4. Use according to any of claims 1 to 3, of FGF-2 mutants corresponding to FGF-2 represented by SEQ ID NO: 4, or by SEQ ID NO: 6, wherein the region delimited by the amino acids located from positions 44 to 61 contains at least one mutation as defined in claim 2.

5. Use according to claims 1 to 4, of FGF-2 mutants corresponding to FGF-2 represented by SEQ ID NO: 4, or by SEQ ID NO: 6, wherein at least one of the amino acids located in positions 44, 48, 52, 54, 55, 58, 61, 65, 68, 69, 71, and 73, is mutated.

6. Use according to any of claims 1 to 5, of FGF-2 mutants corresponding to FGF-2 represented by SEQ ID NO: 4, or by SEQ ID NO: 6, and comprising at least one of the following mutations : H44L, R48T, V52T, E54D, K55R, P58Q, K61Q, Q65S, E68S, R69V, V71E, and S73Y, said mutants being represented by SEQ ID NO: 8, or by SEQ ID NO: 20, respectively.

7. Use according to any of claims 1 to 5, of :
- the FGF-2 mutant comprising the mutations R48T, V52T, and E54D,
- the FGF-2 mutant comprising the mutations H44L, R48T, V52T, and K55R,
- the FGF-2 mutant comprising the mutations H44L, R48T, V52T, and E54D,
- the FGF-2 mutant comprising the mutations Q65S, E68S, R69V, V71E, and S73Y.

8. Use according to any of claims 1 to 6, of FGF-2 mutants corresponding to FGF-2 represented by SEQ ID NO: 4, or by SEQ ID NO: 6, wherein at least the amino acids in positions 54 and 55 are mutated, such as FGF-2 mutants comprising the mutations E54D and K55R.

9. Use according to claim 8, of FGF-2 mutants corresponding to FGF-2 represented by SEQ ID NO: 4, or by SEQ ID NO: 6, wherein at least the amino acids in positions 54 and 55 are mutated, and wherein at least one of the amino acids located in positions 44, 48, 52, 58, and 61, is mutated, such as FGF-2 mutants comprising the mutations E54D and K55R, and at least one of the following mutations H44L, R48T, V52T, P58Q, and K61Q, and more particularly :
- the FGF-2 mutant comprising the mutations E54D, K55R, and H44L,
- the FGF-2 mutant comprising the mutations E54D, K55R, H44L, and R48T,
- the FGF-2 mutant comprising the mutations E54D, K55R, H44L, R48T, and V52T,
- the FGF-2 mutant comprising the mutations E54D, K55R, H44L, R48T, V52T, P58Q, and K61Q.

10. Use according to claims 8 or 9, of FGF-2 mutants corresponding to chimeric FGF-1/FGF-2 proteins.

11. Use according to claim 10, of the following FGF-2 mutants :
- Nbla2, represented by SEQ ID NO: 10, corresponding to a chimeric FGF-1/FGF-2 protein wherein the amino acids in positions 1 to 43 and 79 to 155 belong to bovine FGF-2 represented by SEQ ID NO: 4, and the amino acids located in positions 44 to 78 belong to human FGF-1 represented by SEQ ID NO: 14,
- Nb1a1.6, also referred to as HRVEKPK 44/48/52/54/55/58/61 LTIDRQQ, represented by SEQ ID NO: 12, corresponding to a chimeric FGF-1/FGF-2 protein wherein the amino acids in positions 1 to 43 belong to bovine FGF-2 represented by SEQ ID NO: 4, the amino acids located in positions 44 to 61 belong to human FGF-1 represented by SEQ ID NO: 14, and the amino acids located in positions 62 to 155 belong to human FGF-2 represented by SEQ ID NO: 6.

12. Use according to any of claims 1 to 11, of FGF-2 mutants corresponding to FGF-2 represented by SEQ ID NO: 4, or by SEQ ID NO: 6, wherein the region delimited by the amino acids located from positions 109 to 126 contains at least one mutation as defined in claim 2.

13. Use according to claim 12, of FGF-2 mutants corresponding to FGF-2 represented by SEQ ID NO: 4, or by SEQ ID NO: 6, wherein at least one of the amino acids located in positions 109, 111, 116, 118, 120, 121, 122, 124, and 126, is mutated.

14. Use according to claim 12 or 13, of FGF-2 mutants corresponding to FGF-2 represented by SEQ ID NO: 4, or by SEQ ID NO: 6, and comprising at least one of the following mutations: S109E, N111H, R116T, R118K, Y120H/A/E, S/T121A/E/K, S122E/K/N, Y124F, and A126G, said mutants being represented by SEQ ID NO: 16, or by SEQ ID NO: 22, respectively.

15. Use according to any of claims 12 to 14, of FGF-2 mutants corresponding to FGF-2 represented by SEQ ID NO: 4, or by SEQ ID NO: 6, and comprising the following mutations: S109E, N111H, Y120H/A/E, S/T121A/E/K, S122E/K/N, Y124F, and A126G.

16. Use according to any of claims 12 to 15, of the FGF-2 mutant represented by SEQ ID NO: 18, corresponding to a chimeric FGF-1/FGF-2 protein wherein the amino acids in positions 1 to 108 and 127 to 155 belong to human FGF-2 represented by SEQ ID NO: 6, and the amino acids located in positions 109 to 126 belong to human FGF-1 represented by SEQ ID NO: 14.

17. FGF-2 derived proteins chosen among the following FGF-2 mutants :
- FGF-2 mutants corresponding to FGF-2 represented by SEQ ID NO: 4, or by SEQ ID NO: 6, and comprising at least one of the following mutations: H44L, R48T, V52T, E54D, K55R, P58Q, K61Q, Q65S, E68S, R69V, V71E, and S73Y, said mutants being represented by SEQ ID NO: 8, or by SEQ ID NO: 20, respectively, and more particularly:
· the FGF-2 mutant comprising the mutations R48T, V52T, and E54D,
· the FGF-2 mutant comprising the mutations H44L, R48T, V52T, and K55R,
· the FGF-2 mutant comprising the mutations H44L, R48T, V52T, and E54D,
· the FGF-2 mutant comprising the mutations Q65S, E68S, R69V, V71E, and S73Y.
- FGF-2 mutants corresponding to FGF-2 represented by SEQ ID NO: 4, or by SEQ ID NO: 6, wherein at least the amino acids in positions 54 and 55 are mutated, such as FGF-2 mutants comprising the mutations E54D and K55R,
- FGF-2 mutants corresponding to FGF-2 represented by SEQ ID NO: 4, or by SEQ ID NO: 6, wherein at least the amino acids in positions 54 and 55 are mutated, and wherein at least one of the amino acids located in positions 44, 48, 52, 58, and 61, is mutated, such as FGF-2 mutants comprising the mutations E54D and K55R, and at least one of the following mutations H44L, R48T, V52T, P58Q, and K61Q, and more particularly :
· the FGF-2 mutant comprising the mutations E54D, K55R, and H44L,
· the FGF-2 mutant comprising the mutations E54D, K55R, H44L, and R48T,
· the FGF-2 mutant comprising the mutations E54D, K55R, H44L, R48T, and V52T,
· the FGF-2 mutant comprising the mutations E54D, K55R, H44L, R48T, V52T, P58Q, and K61Q,
- Nbla2, represented by SEQ ID NO: 10, corresponding to a chimeric FGF-1/FGF-2 protein wherein the amino acids in positions 1 to 43 and 79 to 155 belong to bovine FGF-2 represented by SEQ ID NO: 4, and the amino acids located in positions 44 to 78 belong to human FGF-1 represented by SEQ ID NO: 14,
- Nb1a1.6, also referred to as HRVEKPK 44/48/52/54/55/58/61 LTIDRQQ, represented by SEQ ID NO: 12, corresponding to a chimeric FGF-1/FGF-2 protein wherein the amino acids in positions 1 to 43 belong to bovine FGF-2 represented by SEQ ID NO: 4, the amino acids located in positions 44 to 61 belong to human FGF-1 represented by SEQ ID NO: 14, and the amino acids located in positions 62 to 155 belong to human FGF-2 represented by SEQ ID NO: 6,
- FGF-2 mutants corresponding to FGF-2 represented by SEQ ID NO: 4, or by SEQ ID NO: 6, and comprising at least one of the following mutations : S109E, N111H, R116T, R118K, Y120H/A/E, S/T121A/E/K, S122E/K/N, Y124F, and A126G, said mutants being represented by SEQ ID NO: 16, or by SEQ ID NO: 22, respectively,
- FGF-2 mutants corresponding to FGF-2 represented by SEQ ID NO: 4, or by SEQ ID NO: 6, and comprising the following mutations : S109E, N111H, Y120H/A/E, S/T121A/E/K, S122E/K/N, Y124F, and A126G,
- the FGF-2 mutant represented by SEQ ID NO: 18, corresponding to a chimeric FGF-1/FGF-2 protein wherein the amino acids in positions 1 to 108 and 127 to 155 belong to human FGF-2 represented by SEQ ID NO: 6, and the amino acids located in positions 109 to 126 belong to human FGF-1 represented by SEQ ID NO: 14.

18. Nucleotide sequences encoding FGF-2 derived proteins as defined in claim 17.

19. Vectors or plasmids containing nucleotide sequences as defined in claim 18.

20. Host cells transformed with vectors as defined in claim 19.

21. Process for the preparation of FGF-2 derived proteins as defined in claim 17, comprising the culturing of host cells in an appropriate medium, and the purification of said FGF-2 derived proteins produced.

22. Biomaterials or medical devices coated with basic fibroblast growth factor (FGF-2) derived proteins chosen among FGF-2 mutants which are unable to interact specifically with the translokin represented by SEQ ID NO: 2, i.e. having a binding level to said translokin less than 40%, said FGF-2 mutants being such that they are defective in intracellular trafficking and in stimulation of proliferation of endothelial cells, and that their tubulogenesis activity is preserved, for the preparation of biomaterials or medical devices chosen among medical prostheses, such as endovascular stents, coated endoprostheses or bypass grafts.

23. Biomaterials, or medical devices according to claim 22, coated with basic fibroblast growth factor (FGF-2) derived proteins as defined in claim 17.

24. Biomaterials or medical devices according to claim 22 or 23, chosen among endovascular prostheses, such as stents used for inhibiting restenosis or stenosis following angioplasty.
